(19) Europäisches Patentamt
European Patent Office
Office européen des brevets

(11) **EP 3 032 430 A1**

(12) **EUROPEAN PATENT APPLICATION**

(43) Date of publication:
**15.06.2016 Bulletin 2016/24**

(51) Int Cl.:
*G06F 17/18* (2006.01)     *A61B 5/145* (2006.01)
*A61B 5/1495* (2006.01)     *G01N 21/27* (2006.01)
*G01N 21/35* (2014.01)

(21) Application number: **15187881.6**

(22) Date of filing: **01.10.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **03.10.2014 JP 2014204890**

(71) Applicant: **Seiko Epson Corporation Tokyo 163 (JP)**

(72) Inventors:
• **Kurasawa, Hikaru**
  **Nagano, 392-8502 (JP)**
• **Arai, Yoshifumi**
  **Nagano, 392-8502 (JP)**

(74) Representative: **Hoffmann Eitle Patent- und Rechtsanwälte PartmbB Arabellastraße 30 81925 München (DE)**

(54) **TARGET COMPONENT CALIBRATION DEVICE, ELECTRONIC DEVICE, AND TARGET COMPONENT CALIBRATION METHOD**

(57)     A target component calibration device includes a mixing coefficient calculating section that calculates mixing coefficients of target components regarding a test object based on observational data regarding the test obj ect and calibration data, and a target component content calculating section that calculates the content of target components based on the mixing coefficients calculated by the mixing coefficient calculating section and a simple regression equation representing the relationship between the content and the mixing coefficients corresponding to the target components. The target component content calculating section adjusts at least one of two constants of the simple regression equation depending on a measurement condition when the observational data is obtained.

FIG. 7

**Description**

BACKGROUND

1. Technical Field

The invention relates to a technology for calculating the content of target components of a test object from observational data regarding the test object.

2. Related Art

JP-A-2008-5920 discloses a technology for calibrating the concentration of glucoses in a test object. In the related art, in order to increase the calibration precision of seasonal or physiological variations, measurement data sets of multiple invasive measurements and non-invasive measurements are collected for a long period of time during which seasons or temperature changes, and a calibration model which is based on a multiple regression analysis and uses the measurement data sets as calibration data is created based on the calibration data. In the related art, the measurement data sets of the invasive measurements and non-invasive measurements are regularly collected, and the calibration is regularly performed while updating the calibration data. In so doing, the calibration precision of the seasonal and physiological variations is increased.

However, in the related art, since an analysis method based on the multiple regression analysis is used, it is difficult to extract only target components. Thus, in order to cope with a difference in biological condition, a plurality of sample data items having different conditions needs to be prepared, and a calibration regression equation needs to be created. Thus, since the plurality of sample data items is collected for each test object, effort and time are required, or cumbersome calibration is required. Accordingly, there is a need for a technology capable of more easily performing the calibration with high precision.

SUMMARY

An advantage of some aspects of the invention is to solve at least a part of the problems described above, and the invention can be implemented as the following forms or application examples.

(1) An aspect of the invention provides a target component calibration device that calculates the content of target components of a test object. The device includes: a mixing coefficient calculating section that calculates mixing coefficients of the target components regarding the test object based on the calibration data and the observational data regarding the test object; and a target component content calculating section that calculates the content of target components based on the simple regression equation representing the relationship between the content and the mixing coefficients corresponding to the target components, and the mixing coefficients calculated by the mixing coefficient calculating section, in which the target component content calculating section adjusts at least one of two constants of the simple regression equation depending on a measurement condition when the observational data is obtained.

According to the target component calibration device, since the simple regression equation is adjusted depending on the measurement condition when the observational data is obtained, it is possible to increase calibration precision depending on the measurement condition, and it is possible to easily perform the calibration since it is not necessary to perform the measurement several times.

In one embodiment, the target component calibration device includes a test object observational data obtaining section that obtains the observational data regarding the test object; a calibration data obtaining section that obtains calibration data including independent components corresponding to the target components and a calibration simple regression equation; a mixing coefficient calculating section that calculates mixing coefficients of the target components of the test object based on the observational data regarding the test obj ect and the calibration data; and a target component content calculating section that calculates the content of target components based on the mixing coefficients obtained by the mixing coefficient calculating section and the simple regression equation representing the relationship between the content and the mixing coefficients corresponding to the target components. The target component content calculating section adjusts two constants of the simple regression equation depending on the measurement condition when the observational data is obtained.

According to the target component calibration device, it is possible to calculate the content of target components of the test object with high precision by simply obtaining one observational data item regarding the test object.

(2) In the target component calibration device, the measurement condition may be a measurement environment, and the target component content calculating section may adjust at least one of the two constants of the simple regression equation depending on the measurement environment. In such a configuration, it is possible to increase the calibration precision by adjusting the simple regression equation depending on the measurement environment. The measurement environment is an environmental state where the measurement is performed. A preferred example of the measurement environment is a temperature of the test object or a temperature in a place where the measurement is performed. Accordingly, when the test object is a person, the measurement environment is the body temperature of the person or a temperature in a place where the person is present.

(3) In the target component calibration device, the measurement condition may be a difference among examinees as the test object, and the target component content calculating section may adjust at least one of the two constants of the simple regression equation depending on the difference among the examinees. In such a configuration, it is possible to increase the calibration precision by adjusting the simple regression equation depending on the examinee.

The invention may be implemented in various forms other than the aforementioned form, and may be implemented by, for example, an electronic device including the aforementioned device, computer programs for implementing the functions of the respective units of the aforementioned device, and a non-transitory storage medium that temporarily stores the computer programs.

## BRIEF DESCRIPTION OF THE DRAWINGS

The invention will be described with reference to the accompanying drawings, wherein like numbers reference like elements.

Fig. 1 is an explanatory diagram showing the outline of a calibration curve creation process using independent component analysis.
Fig. 2 is an explanatory diagram showing the outline of a calibration process of target components.
Figs. 3A and 3B are graphs showing a first adjustment example of a calibration curve.
Figs. 4A and 4B are graphs showing a second adjustment example of the calibration curve.
Fig. 5 is a flowchart of the calibration curve creation process.
Fig. 6 is an explanatory diagram showing a computer used in the calibration curve creation process.
Fig. 7 is a functional block diagram of a device used in the calibration curve creation process.
Fig. 8 is a functional block diagram showing an example of an internal configuration of an independent component matrix calculating section.
Fig. 9 is an explanatory diagram that schematically shows a measurement data set DS1.
Fig. 10 is a flowchart of a mixing coefficient estimating process.
Fig. 11 is an explanatory diagram for describing an estimation mixing matrix ^A.
Fig. 12 is a flowchart of a regression equation calculating process.
Fig. 13 is a functional block diagram of a device used in a target component calibration process.
Fig. 14 is a flowchart of the target component calibration process.

## DESCRIPTION OF EXEMPLARY EMBODIMENTS

Hereinafter, exemplary embodiments of the invention will be described in the following order.

A. Outline of Calibration Curve Creation Process and Calibration Process:
B. Adjustment Example of Calibration Curve:
C. Calibration Curve Creation Method:
D. Calibration Method of Target component:
E. Various Algorithms and Influences thereof:
F. Modification Examples:

In the present exemplary embodiment, the following abbreviations are used.

· ICA: Independent Component Analysis
· SNV: Standard Normal Variate Transformation
· PNS: Project on Null Space
· PCA: Principal Components Analysis
· FA: Factor Analysis

A. Outline of Calibration Curve Creation Process and Calibration Process:

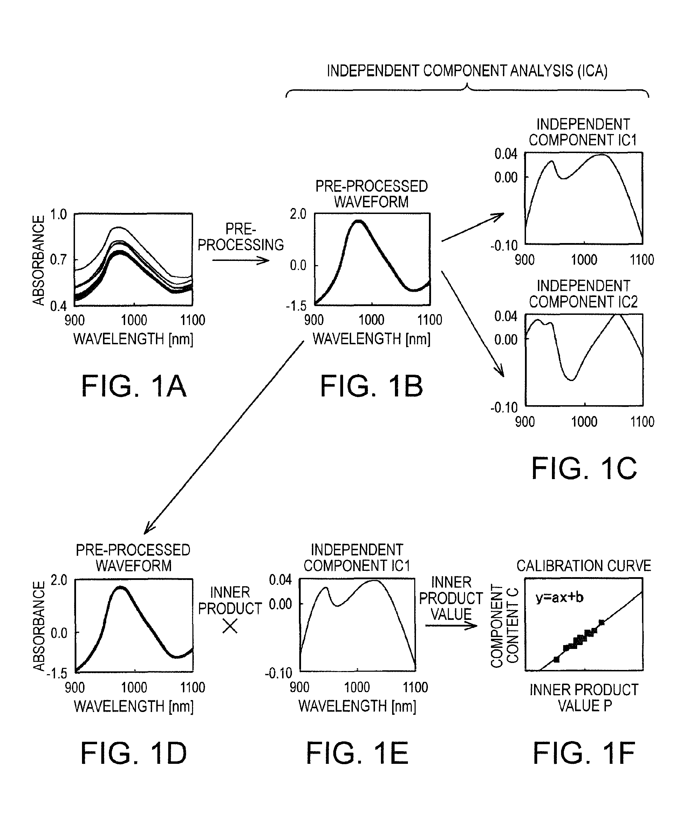

Fig. 1 is an explanatory diagram showing the outline of a calibration curve creation process using an independent component analysis. (A) of Fig. 1 shows an example of observational data (referred to as "measurement data") on a plurality of samples. The observational data is spectral absorbance, and can be obtained by performing spectrometry on a sample including a plurality of chemical components such as glucose. As the plurality of samples used in the calibration curve creation process, the sample of which the content of target components (for example, glucoses) is known is used. Instead, the content of target components included in the plurality of samples may be measured in an analyzing device.

When a calibration curve is created, variation or noise included in the observational data are reduced ((B) of Fig. 1) by performing a pre-processing on the observational data. For example, as the pre-processing, a first pre-processing including normalization of the observational data, and a second pre-processing including whitening are performed. In the first pre-processing, it is preferable that the project on null space is performed in order to reduce an influence by various variation factors (such as a state of the sample or a change of a measurement environment) of the observational data. Subsequently, a plurality of independent components IC1, IC2, ... ((C) of Fig. 1) is obtained by performing the independent component analysis process on the pre-processed observational data. The independent components IC1, IC2, ... are data items that respectively correspond to substance components included in the respective samples, and are statistically independent components from each other. The observational data items of the respective samples may be reproduced by linearly combining the independent components IC1, IC2, .... In (C) of Fig. 1, although only two independent components IC1 and IC2 are illustrated, the number of independent components is appropriately set to be any number of 2 or more. In the description of the exemplary embodiment, the term "target component" means a substance or chemical component included in the sample, and the term "independent component" means data having the same data length as the observational data regarding the sample.

Thereafter, as shown in (D) to (F) of Fig. 1, an inner product of the independent component (for example, IC1) and the pre-processed observational data is calculated. The observational data of (D) of Fig. 1 is the same as that of (B) of Fig. 1. When the inner product of one observational data item and one independent component IC1 is calculated, one inner product value is obtained for the observational data. Accordingly, when inner products of the plurality of observational data items and the same independent component IC1 are calculated, a plurality of inner product values with respect to the independent component IC1 is obtained for the plurality of samples. (F) of Fig. 1 is a diagram plotted by representing an inner product value P for the plurality of samples as a horizontal axis and the known content C of target components included in the plurality of samples as a vertical axis. If the independent component IC1 used in the inner product is an independent component corresponding to the target component, the inner product value P and the content C of target components of the respective samples have a strong correlation, as shown in (F) of Fig. 1. Thus, of the plurality of independent components IC1, IC2, ... obtained in (C) of Fig. 1, the independent component representing the strongest correlation may be selected as the independent component corresponding to the target component. In the example of Fig. 1, the independent component IC1 is an independent component corresponding to a calibrating target component (for example, glucose). The calibration curve is represented as a straight line which is given as simple regression equation $C = uP + v$ and is a plot of (F) of Fig. 1. Since the inner product value P is a value which is proportional to the content of the independent components IC1 in the respective samples, the inner product value is also referred to as a "mixing coefficient".

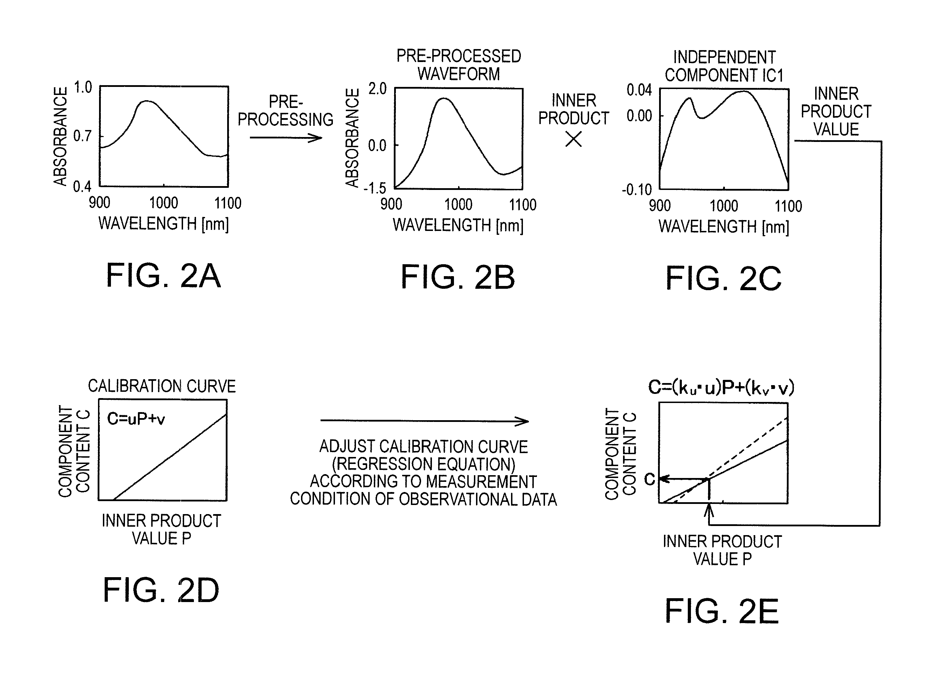

Fig. 2 is an explanatory diagram showing the outline of a calibration process of the target component using the calibration curve. The calibration process is performed using the independent component IC1 ((E) of Fig. 1) of the target component obtained in the calibration curve creation process shown in Fig. 1 and the calibration curve ((F) of Fig. 1). In the calibration process, observational data on a sample of which the content of target components is not known is obtained ((A) of Fig. 2). Subsequently, the pre-processing is performed on the observational data ((B) of Fig. 2). It is preferable that this pre-processing is the same pre-processing used at the time of creation of the calibration curve. The inner product value P is calculated for the observational data by calculating the inner product of the pre-processed observational data and the independent component IC1 ((B) of Fig. 2). The content C of target components can be determined by applying the inner product value P to the calibration curve.

In the present exemplary embodiment, the simple regression equation of the calibration curve is adjusted depending on a measurement condition of observational data on a test object ((E) of Fig. 2). For example, it is possible to adjust the simple regression equation according to the following equation.

$$C = (k_u \cdot u) \, P + (k_v \cdot v) \qquad \qquad \ldots (1)$$

Here, C is the content of target components, P is an inner product value (mixing coefficient), u and v are constants of the non-adjusted simple regression equation, $k_u$ and $k_v$ are adjustment coefficients of the constants u and v. The adjustment coefficients $k_u$ and $k_v$ are previously determined as appropriate values depending on the measurement condition and are set within the calibration device before the target components are calibrated.

As stated above, it is possible to more precisely perform the calibration by using the adjusted calibration curve.

It is possible to easily increase calibration precision by adjusting the calibration curve without collecting a plurality of sample data items for each test object.

For example, as the measurement condition, a measurement environment such as measurement temperature (temperature of the test object or temperature in a place where the measurement is performed), humidity or atmospheric pressure, and a difference between examinees as the test object may be considered. When the calibration curve is adjusted based on the physical measurement condition (measurement environment) such as temperature, humidity and atmospheric pressure, it is preferable that the values of the adjustment coefficients $k_u$ and $k_v$ are previously determined depending on the measurement condition, and the determined values are stored. When the difference between the examinees as the test object is considered as a difference in the measurement condition, it is preferable that the adjustment coefficients $k_u$ and $k_v$ of Equation (1) are previously determined for each test object. In place of determining the adjustment coefficients $k_u$ and $k_v$, the adjusted constants $(k_v \cdot u)$ and $(k_v \cdot v)$ may be previously determined and the determined values may be stored. It should be noted that appropriate constants $(k_v \cdot u)$ and $(k_v \cdot v)$ of the simple regression equation of the calibration are previously determined depending on the measurement condition in both cases. The calibration curve creation process of Fig. 1 or the calibration process of Fig. 2 will be described in detail below.

In the calibration method using the multiple regression analysis described in the related art, since independent separation is not performed on the components unlike the independent component analysis, it is necessary to create the regression equation of the calibration curve by using a data set including all the differences in the measurement conditions. For example, when a blood-sugar level of a human body is calibrated, since an individual difference is included in the difference in the measurement condition, if calibration data is not collected for each examinee for a long period of time, it is difficult to use this calibration method. Since the differences in the measurement conditions represented by the observational data are expressed by the calibration regression equation of the multiple regression, the number of parameters (coefficients) included in the regression equation also increases spontaneously. Since information items on the target components are present in the respective explanatory variables in a distributed manner, if the measurement condition is changed, a contribution method of the target component to all the explanatory variables is changed. For this reason, all the coefficients included in the regression equation of the calibration curve need to be adjusted, and since at least data sets having the number corresponding to the number of coefficients included in the regression equation are needed in order to adjust the calibration curve depending on the difference in the measurement condition, it is necessary to perform a complicated and troublesome process.

Here, for example, when the number of explanatory variables being 7 is considered in the multiple regression analysis, the calibration regression equation in this case is given by the following equation.

$$Y = a_1 X_1 + a_2 X_2 + \ldots + a_7 X_7 + b$$

Here, Y is an objective variable, $X_i$ (i = 1 to 7) is an explanatory variable, and $a_i$ (i = 1 to 7) and b are coefficients.

In this case, a system of equations with 8 unknowns is solved in order to adjust 8 coefficients $a_i$ and b, and thus, it is necessary to use the data as many as 8 objective variables and 56 explanatory variables.

$$Y_1 = a_1 X_{11} + a_2 X_{12} + \ldots + a_7 X_{17} + b$$

$$\ldots\ldots$$

$$Y_8 = a_1 X_{81} + a_2 X_{82} + \ldots + a_7 X_{87} + b$$

Meanwhile, in the exemplary embodiment shown in Fig. 2, since the simple regression equation C = uP + v is used as the calibration curve, in order to adjust the constants u and v of the simple regression equation, two sets of objective variables C and explanatory variables P may be used. Accordingly, it is possible to calibrate the calibration curve with a small number of measurements unlike the related art.

B. Adjustment Example of calibration curve:

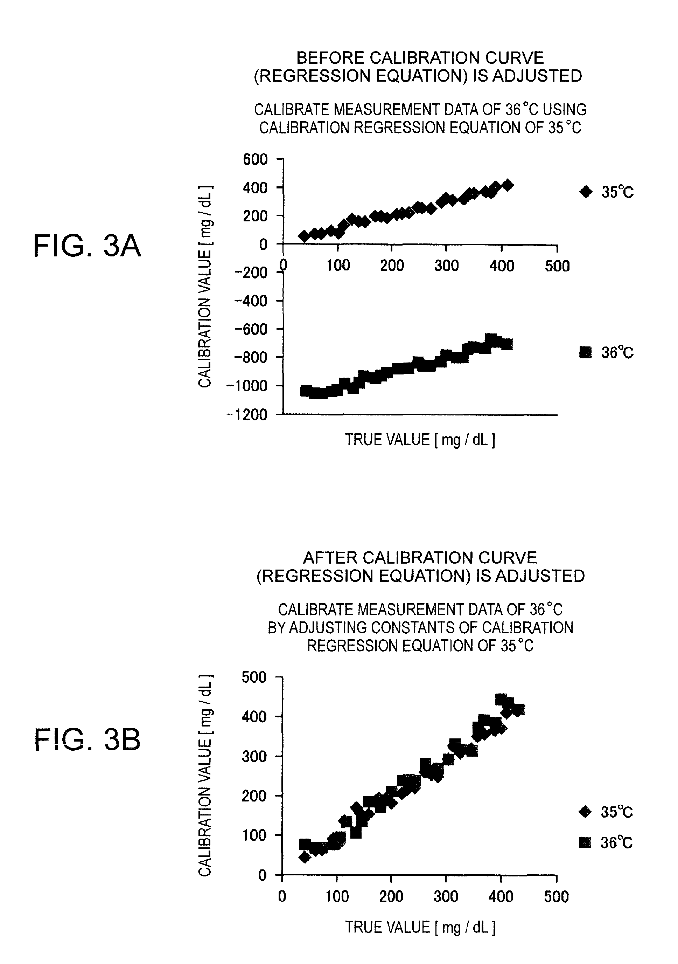

Figs. 3A and 3B are graphs showing a first adjustment example of the calibration curve. In this example, a glucose aqueous solution is used as the test object. An optical spectrum of the glucose aqueous solution is measured when different temperatures, 35°C and 36°C, are the measurement condition. The calibration is performed on measurement data of 36°C by using calibration data (the independent component and the calibration regression equation) created using measurement data of 35°C. A specific procedure is as follows.

(1a) Glucose aqueous solutions having different concentrations at a level of 28 between 40 to 410 mg/dL are prepared.
(1b) An optical spectrum of the glucose aqueous solution of 35°C is measured by a spectroscopic measurement device, and sample data is obtained.
(1c) The pre-processing, the independent component analysis process and the derivation of the calibration regression equation are performed on the sample data according to the procedure (Fig. 1) of the calibration curve creation process, and calibration data (the independent component and the calibration regression equation $C = uP + v$) are created.
(1d) The glucose aqueous solution is controlled to 36°C, the optical spectrum is similarly measured, and test data is obtained.
(1e) The calibration regression equation is adjusted by multiplying the constant u of the calibration regression equation by the proper coefficient $k_u$ in accordance with a temperature difference between the aqueous solutions (the adjustment coefficient $k_v$ of the constant v is equal to 1).
(1f) The calibration precision is obtained by performing the calibration on test data by using the adjusted calibration regression equation.

The calibration regression equation obtained in step (1c) is as follows.

$$C = 13548.73P - 246387.33$$

When the test data regarding the glucose aqueous solution of 36°C is calibrated by using the calibration regression equation and the independent component as it is, the calibration precision (a prediction standard deviation SEP between an actual value and a calibrated value) is 1103.9 mg/dL, and the calibration precision is extremely inadequate (Fig. 3A).

Meanwhile, in step (1e), a new calibration regression equation is obtained as follows by multiplying the constant u of the calibration regression equation by the coefficient $k_u = 1.00456$.

$$C = 13610.67P - 246387.33$$

When the calibration is performed on the test data of 36°C by using the calibration regression equation, the calibration precision is improved as SEP = 24.4 mg/dL (Fig. 3B)).

The coefficient $k_u = 1.00456$ used in the adjustment of the constant u in step (1e) is obtained by selecting an inner product value P1 (mixing coefficient) corresponding to one glucose concentration $C_1$ from the test data of 36°C and substituting the selected inner product value in the following equation obtained by changing the coefficient $k_v$ to 1 in Equation (1).

$$k_u = (C_1 - v) / (u \cdot P_1)$$
$$= (C_1 + 246387.33) / (13610.67 P_1) \quad \cdots (2)$$

In the first adjustment example, it can be seen that the calibration can be performed with high precision irrespective of the difference in the measurement condition (measurement temperature). It is preferable that a sensor that measures the measurement condition (for example, temperature) is provided at a target component calibration device or the relationship between the measurement condition and the adjustment coefficients $k_u$ and $k_v$ (or the relationship between the measurement condition and the adjusted constants $(k_u \cdot u)$ and $(k_v \cdot v)$ ) is previously stored in the target component calibration device such that the target component calibration device can adjust the calibration curve depending on the measurement condition such as the measurement temperature. In so doing, when the sensor measures the measurement condition, the target component calibration device can perform the calibration with high precision by using the calibration curve defined by the constants $(k_u \cdot u)$ and $(k_v \cdot v)$ appropriate for the measurement condition.

In the first adjustment example, although the adjustment coefficient $k_v$ of the constant v indicating an intercept of the calibration curve is equal to 1, a more precise calibration curve is obtained by setting the adjustment coefficient $k_v$ to be a value other than 1 depending on the measurement condition in some cases. In other words, it is possible to obtain a more precise calibration curve by adjusting at least one of two constants u and v of the simple regression equation of the calibration curve depending on the measurement condition.

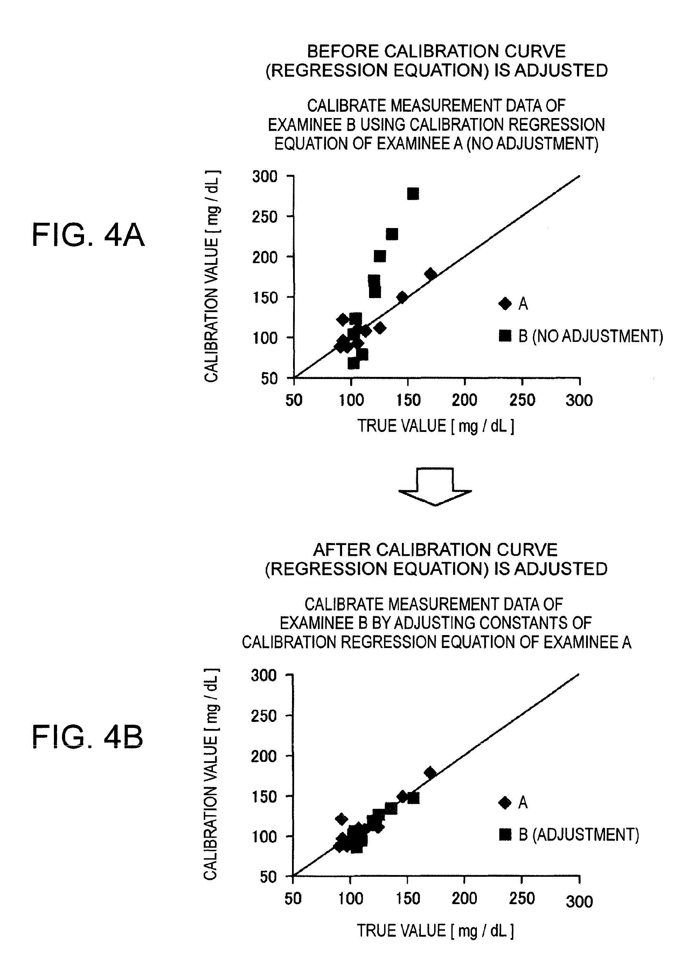

Figs. 4A and 4B are graphs showing a second adjustment example of the calibration curve. In the second adjustment example, the calibration curve is adjusted when the human body is used as the test object and the blood-sugar level (glucose concentration) is calibrated from optical spectrum data regarding the human body. In the second adjustment example, the difference in the measurement condition is an individual difference between examinees. Specifically, the optical spectrum of the human body (for example, hand) and the blood-sugar level through blood drawn are measured for an examinee A and an examinee B ten times. The calibration data (the independent component and the calibration regression equation) is first created using measurement data regarding the examinee A based on these measurement data items. Subsequently, the precision thereof is checked by adjusting the created calibration repression equation and performing the calibration of the blood-sugar level on the measurement data regarding the examinee B. A specific procedure is as follows.

(2a) The optical spectrum of the human body of the examinee A is measured in the spectroscopic measurement device, and the blood-sugar level of the blood obtained through blood drawn is measured. These measurements are performed ten times.

(2b) The measurement spectrum of the examinee A is obtained as sample data.

(2c) The pre-processing, the independent component analysis process and the deviation of the calibration regression equation are performed on the sample data according to the procedure (Fig. 1) of the calibration curve creation process, and the calibration data (independent component and the calibration regression equation $C = uP + v$) are created.

(2d) The test data is obtained by measuring the optical spectrum of the human body of the examinee B in the spectroscopic measurement device and measuring the blood-sugar level of the blood obtained through blood drawn.

(2e) The calibration regression equation is adjusted by multiplying the constants u and v of the calibration regression equation by the proper coefficients $k_u$ and $k_v$ in accordance with the examinee B.

(2f) The calibration precision (SEP) is obtained by performing the calibration on the test data by using the adjusted calibration regression equation.

The calibration regression equation obtained in step (2c) is as follows.

$$C = -91.20P - 1358.64$$

When the blood-sugar level of the test data regarding the examinee B is calibrated using the calibration regression equation and the independent component as it is, the calibration precision is SEP = 62.5 mg/dL, and the calibration precision is inadequate (Fig. 4A).

Meanwhile, in step (2e), the following new calibration regression equation is obtained by respectively multiplying the constants u and v of the calibration regression equation by the coefficients $k_u = 0.2141$ and $k_v = 0.2686$.

$$C = -24.50P - 290.84$$

When the calibration of the test data of the examinee B is performed using the calibration regression equation, the calibration precision is improved as SEP = 8.4 mg/dL (Fig. 4B) .

For comparison, calibration data (the independent component and the calibration regression equation) is created from the measurement data regarding the examinee B according to the procedure of Fig. 1. When the measurement data regarding the examinee B is calibrated using the calibration data, the calibration precision is SEP = 7.2 mg/dL. As mentioned above, in the second adjustment example, it can be seen that it is possible to perform the calibration having the same precision as that when the calibration curve created using the measurement data regarding the examinee himself or herself is used by adjusting the calibration curve created using the measurement data items of different examinees. The calibration precision having a SEP of 7 to 8 mg/dL is a value close to the precise value when the blood drawn is analyzed in a highly precise analyzing device. That is, in the second adjustment example, it is possible to measure an extremely highly precise blood-sugar level with similar precision to the non-invasive measurement through a non-invasive measurement such as the optical spectrum measurement using the human body as a target.

In step (2e), the coefficient $k_u = 0.2141$ and $k_v = 0.2686$ used during the adjustment of the constants u and v are obtained by selecting the inner product values P1 and P2 (mixing coefficients) corresponding to two true values $C_1$ and $C_2$ from the test data of the examinee B and solving a system of equations related to Equation (1).

$$C_1 = (k_u \cdot u)\, P_1 + (k_v \cdot v) \qquad \cdots (3a)$$

$$C_2 = (k_u \cdot u)\, P_2 + (k_v \cdot v) \qquad \cdots (3b)$$

In the second adjustment example, it can be seen that the calibration can be performed with high precision irrespective of the difference between the examinees as the measurement condition. It is preferable that an input unit that inputs an ID of the examinee (a name of the examinee or an unique number of the examinee) is provided in the target component calibration device and the ID of the examinee and the adjustment coefficients $k_u$ and $k_v$ (or the adjusted constants $(k_u \cdot u)$ and $(k_v \cdot v)$) appropriate for the examinee are previously stored in the target component calibration device such that the target component calibration device can adjust the calibration curve depending on the difference between the examinees. In so doing, when the examinee (user) inputs the ID of the examinee in the target component calibration device, the target component calibration device can perform the calibration with high precision by using the calibration curve defined by the constants $(k_v \cdot u)$ and $(k_v \cdot v)$ appropriate for the examinee.

As a method of obtaining the adjustment coefficients $k_u$ and $k_v$ of the constants u and v of the calibration regression equation, it is possible to adopt any method other than the aforementioned method.

C. Calibration Curve Creation Method:

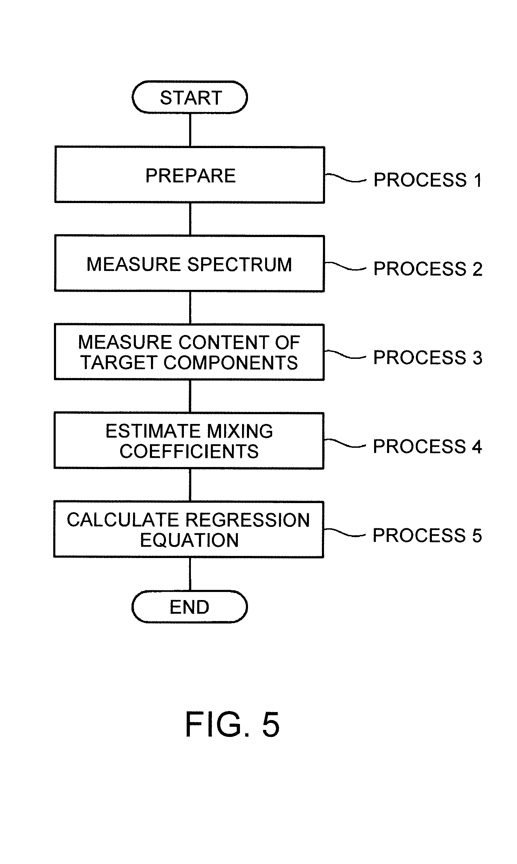

Fig. 5 is a flowchart showing the calibration curve creation method according to the exemplary embodiment of the invention. The calibration curve creation method includes five processes from Process 1 to Process 5. Processes 1 to 5 are performed in sequence. Processes 1 to 5 will be described in sequence.

Process 1

Process 1 is a preparation process, and is performed by an operator. The operator obtains (prepares) a plurality of same kind of samples (for example, a glucose aqueous solution or a human body) of which the target components have different contents. In the present exemplary embodiment, n (n is an integer of 2 or more) samples are used.

Process 2

Process 2 is a spectrum measurement process, and is performed using a spectroscopic measurement device by the operator. The operator measures a spectral reflectance spectrum of each sample by photographing the plurality of samples prepared in Process 1 in the spectroscopic measurement device. The spectroscopic measurement device is a known device that measures the spectrum by transmitting light from a measured body to a spectrometer and receiving the spectrum output from a spectrometer on an imaging surface of an imaging device. The relationship expressed by the following equation is satisfied between the spectral reflectance spectrum and the absorbance spectrum.

$$[\text{Absorbance}] = -\log_{10}[\text{Reflectance}] \quad \dots (4)$$

The measured spectral reflectance spectrum is converted into absorbance spectrum by using Equation (4). Converting the spectral reflectance spectrum into the absorbance spectrum is because linear combination needs to be satisfied for a mixed signal analyzed in the independent component analysis, to be described, and linear combination is satisfied for the absorbance from the Lambert-Beer law. Accordingly, in Process 2, the absorbance spectrum may be measured in place of the spectral reflectance spectrum. As the measured result, absorbance distribution data representing characteristics with respect to a wavelength of the measured body is output. The absorbance distribution data is also called spectrum data.

In place of measuring the spectral reflectance spectrum and the absorbance spectrum in the spectrometer, these spectra may be estimated from other measured values. For example, the sample may be measured using a multiband camera, and the spectral reflectance spectrum or absorbance spectrum may be estimated from the obtained multiband images. For example, as such an estimation method, a method described in JP-A-2001-99710 may be used.

Process 3

Process 3 is a process of measuring the content of the target component, and is performed by the operator.

The operator measures the content (for example, the amount of glucoses) of the target component with the respective samples by performing a chemical analysis on the plurality of samples prepared in Process 1. When the content of target components in the samples prepared in Process 1 is known, Process 3 may be omitted.

Process 4

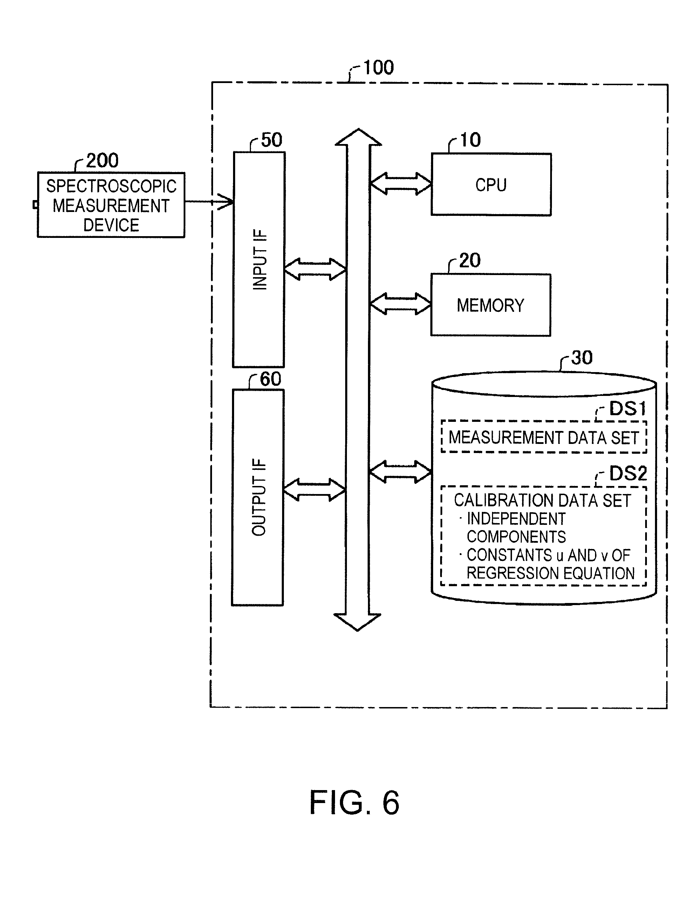

Process 4 is a process of estimating the mixing coefficient, and is typically performed using a computer. Fig. 6 is an explanatory diagram showing a computer 100 used in Processes 4 and 5, to be described, and a peripheral device. The computer 100 is electrically connected to a spectroscopic measurement device 200.

The computer 100 is a known device including a CPU 10 that performs various processes or controls by executing computer programs, a memory 20 (storage section) that is a data saving place, a hard disk drive 30 that stores the computer programs or data, an input interface 50, and an output interface 60.

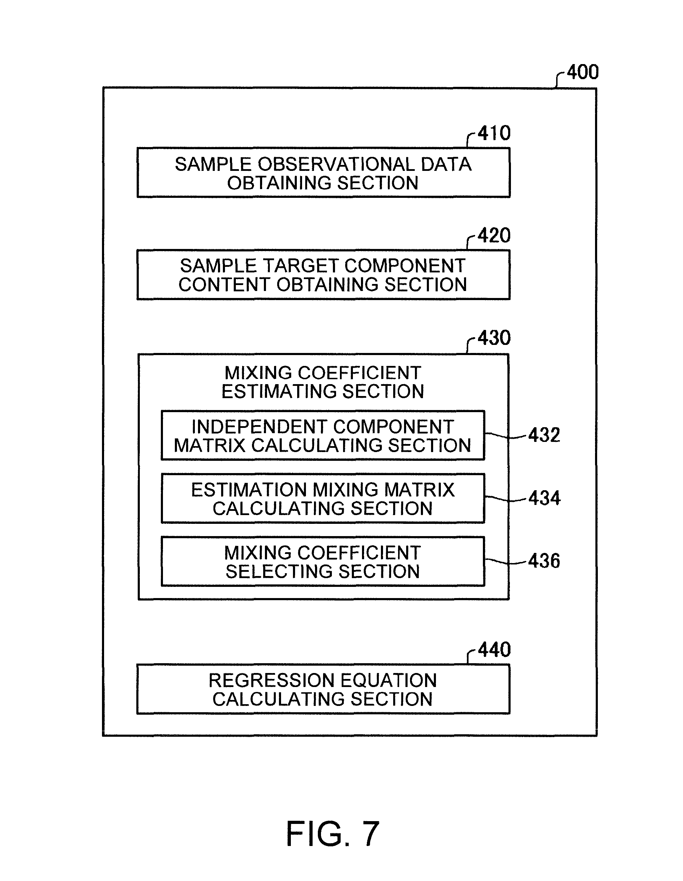

Fig. 7 is a functional block diagram of a device used in Process 4 and Process 5. This device 400 includes a sample observational data obtaining section 410, a sample target component content obtaining section 420, a mixing coefficient estimating section 430, and a regression equation calculating section 440. The mixing coefficient estimating section 430 includes an independent component matrix calculating section 432, an estimation mixing matrix calculating section 434, and a mixing coefficient selecting section 436. The sample observational data obtaining section 410 and the sample target component content obtaining section 420 are implemented by, for example, the CPU 10 of Fig. 6 in cooperation with the input I/F 50 and the memory 20. The mixing coefficient estimating section 430, the independent component matrix calculating section 432, the estimation mixing matrix calculating section 434 and the mixing coefficient selecting section 436 are implemented by, for example, the CPU 10 of Fig. 6 in cooperation with the memory 20. The regression equation calculating section 440 is implemented by, for example, the CPU 10 of Fig. 6 in cooperation with the memory 20. The respective sections may be implemented by other specific devices or hardware other than the computer shown in Fig. 6.

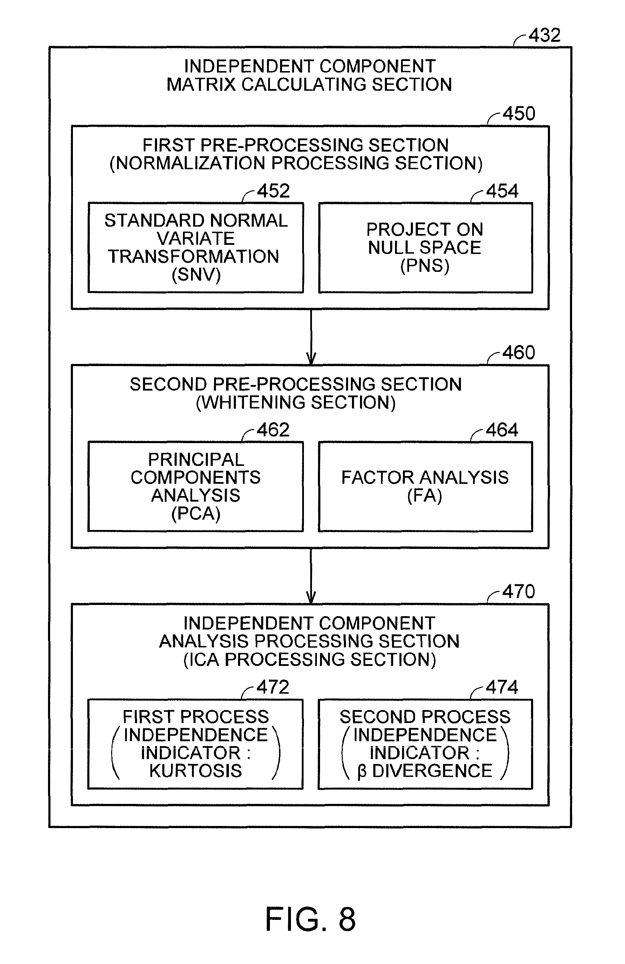

Fig. 8 is a functional block diagram showing an example of an internal configuration of the independent component matrix calculating section 432. The independent component matrix calculating section 432 includes a first pre-processing section 450, a second pre-processing section 460, and an independent component analysis processing section 470. The three processing sections 450, 460 and 470 obtain an independent component matrix (to be described below) by sequentially processing process target data (the absorbance spectra in the present exemplary embodiment). The process contents of the respective sections will be described below.

The spectroscopic measurement device 200 shown in Fig. 6 is used in Process 2. The computer 100 obtains the absorbance spectrum obtained from the spectral distribution measured by the spectroscopic measurement device 200 in Process 2 through the input I/F 50, as the spectral data (corresponding to the sample observational data obtaining section 410 of Fig. 7). The computer 100 calculates the content of target components measured in Process 3 through an operation of the operator using a keyboard (corresponding to the sample target component content obtaining section 420 of Fig. 7).

As a result of obtaining the spectral data and the target component content, the data set (hereinafter, referred to as a "measurement data set") DS1 including the spectral data and the target component content is stored in the hard disk drive 30 of the computer 100.

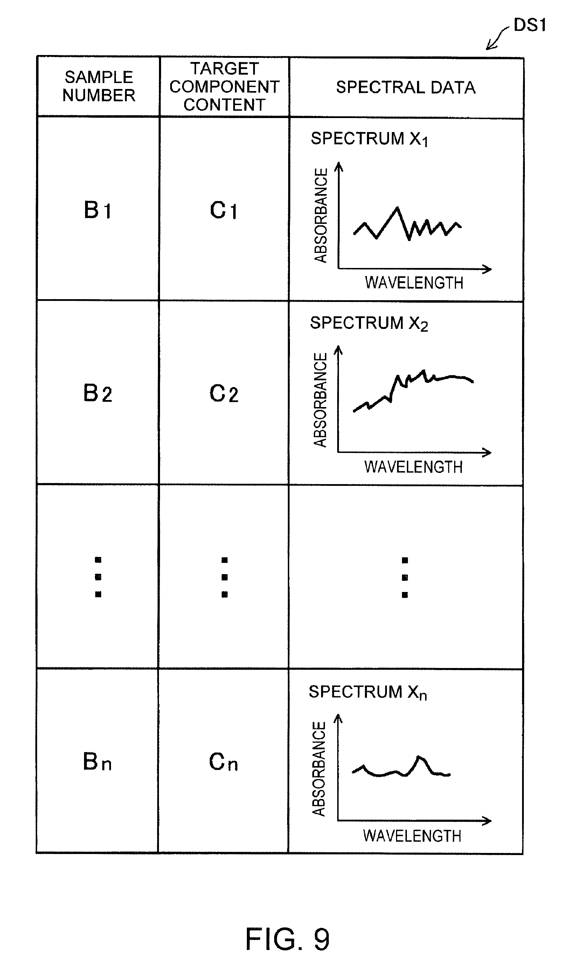

Fig. 9 is an explanatory diagram that schematically shows the measurement data set DS1 stored in the hard disk drive 30. As shown in this drawing, the measurement data set DS1 is a data structure that includes sample numbers $B_1$, $B_2$, ..., and $B_n$ for identifying the plurality of samples prepared in Process 1, target component contents $C_1$, $C_2$, ..., and $C_n$ of the respective samples, and spectral data items $X_1$, $X_2$, ..., and $X_n$ of the respective samples. In the measurement data set DS1, the target component contents $C_1$, $C_2$, ..., and $C_n$ and the spectral data items $X_1$, $X_2$, ..., and $X_n$ are correlated with the sample numbers $B_1$, $B_2$, ..., and $B_n$ so as to determine the sample to which the target component content and the spectral data correspond.

The CPU 10 performs a process of estimating the mixing coefficient which is an operation of Process 4 by loading a predetermined program stored in the hard disk drive 30 to the memory 20 and executing the loaded program. Here, the predetermined program may be downloaded via a network such as the Internet from the outside. In Process 4, the CPU 10 functions as the mixing coefficient estimating section 430 of Fig. 7.

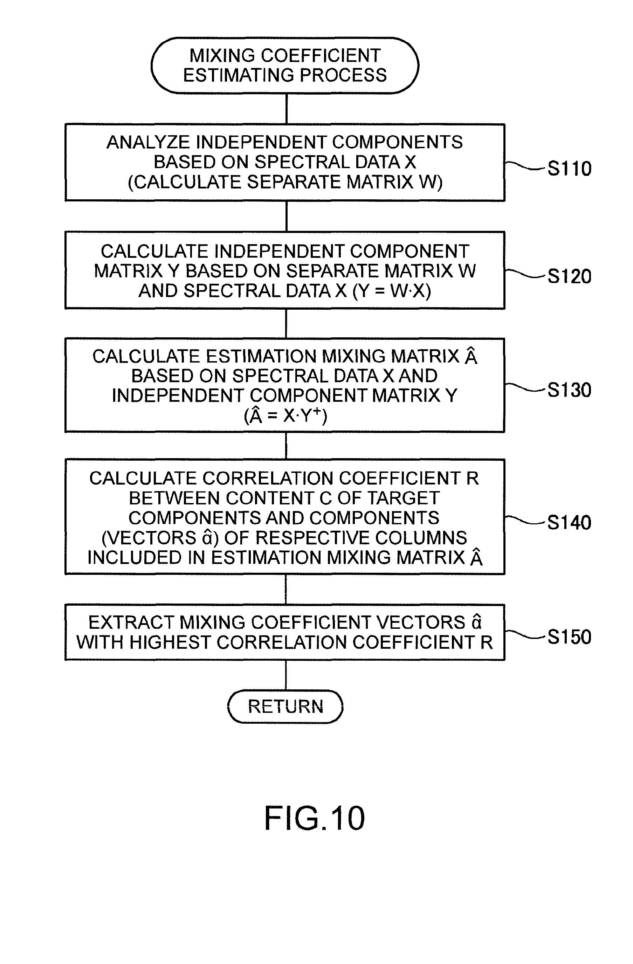

Fig. 10 is a flowchart showing the mixing coefficient estimating process performed by the CPU 10. When the process starts, the CPU 10 performs the independent component analysis (step S110).

The independent component analysis (ICA) is one of multidimensional signal analysis methods, and is a technique of observing a mixed signal in which independent signals overlap each other in some different conditions and separating the independent signals based on the mixed signal. Due to the use of the independent component analysis, the spectral data obtained in Process 2 is regarded as being mixed with m independent components (unknown) including the target component, and thus, it is possible to estimate spectra of the independent components from the spectral data (observational data) obtained in Process 2.

In the present exemplary embodiment, the independent component analysis is performed through processes that are sequentially performed by the three processing sections 450, 460 and 470 shown in Fig. 8. The first pre-processing section 450 may perform the pre-processing using one or both of standard normal variate transformation (SNV) 452 and project on null space (PNS) 454. The SNV 452 is a process of obtaining normalized data of which the average value is 0 and the standard deviation is 1 by subtracting the average value of the processing target data items and dividing the resultant value by the standard deviation. The PNS 454 is a process for removing variation in a baseline included in the processing target data. In the spectral measurement, variation between data items which is called a baseline variation, such as an increase or decrease of the average value of the data items for each measurement data, occurs due to various factors. For this reason, it is preferable that theses various factors are removed before the independent component analysis process is performed. The PNS may be used as the pre-processing capable of removing any baseline variation. For example, the PNS is described in "Extracting Chemical Information from Spectral Data with Multiplicative Light Scattering Effects by optical Path-Length Estimation and Correction (Zeng-Ping Chen, Julian Morris, and Elaine Martin; 2006)".

When the SNV 452 is performed on the spectral data obtained in Process 2 of Fig. 5, it is not necessary to perform the process of the PNS 454. Meanwhile, when the process of the PNS 454 is performed, it is preferable that some normalization processes (for example, SNV 452) are subsequently performed.

As the first pre-processing, processes other than the SNV or the PNS may be performed. It is preferable that some normalization processes are performed in the first pre-processing, but the normalization process may be omitted. Hereinafter, the first pre-processing section 450 is also referred to as a "normalization processing section". The details of the two processes 452 and 454 will be further described below. When the processing target data supplied to the independent component matrix calculating section 432 is normalized data, the first pre-processing may be omitted.

The second pre-processing section 460 may perform the pre-processing using any one of a principal components analysis (PCA) 462 and a factor analysis (FA) 464. As the second pre-processing, a process other than the PCA or the FA may be used. Hereinafter, the second pre-processing section 460 is also referred to as a "whitening processing section". In a general ICA method, dimensional compression of the processing target data and decorrelation are performed as the second pre-processing. By the use of the second pre-processing, since a transformation matrix to be obtained in the ICA is limited to an orthogonal transformation matrix, it is possible to reduce the calculation amount of the ICA. The second pre-processing is called the "whitening", and the PCA is used in many cases. However, when a random noise is included in the processing target data, the PCA is influenced by the noise, and an error may occur in the result. Thus, in order to reduce the influence of the random noise, it is preferable that the whitening is performed using the FA having robustness against the noise in place of the PCA. The second pre-processing section 460 of Fig. 8 may perform the whitening by selecting any one of the PCA and the FA. The details of the two processes 462 and 464 will be further described below. The whitening process may be omitted.

The independent component analysis processing section (ICA processing section) 470 estimates independent component spectra by performing the ICA on the spectral data on which the first pre-processing and the second pre-processing have been performed. The ICA processing section 470 may perform the analysis using any one of fist processing 472 using kurtosis as an independence indicator, and second processing 474 using $\beta$ divergence as an independence indicator. In general, the ICA uses higher-order statistics indicating the independence between the data items separated using an indicator for separating the independent components, as the independence indicator. The kurtosis is a typical independence indicator. However, when an outlier such as a spike noise is included in the processing target data, statistics including the outlier is calculated as the independence indicator. For this reason, an error occurs between the original statistics and the calculated statistics of the processing target data, and degradation in separation precision is caused in some cases. Thus, in order to reduce the influence by the outlier included in the processing target data, it is preferable that an independence indicator that is not easily influenced by the outlier included in the processing target data is used. As the independence indicator having such characteristics, the $\beta$ divergence may be used. The details of the kurtosis and the $\beta$ divergence will be further described below. As the independence indicator of the ICA, an indicator other than the kurtosis and the $\beta$ divergence may be used.

Next, the typical processing details of the independent component analysis will be described. It is assumed that spectra S (hereinafter, these spectra are simply referred to as "unknown components") of m unknown components (sources) are given as vectors of Equation (5) and the n spectral data items X obtained in Process 2 are given as vectors of Equation (6). The respective elements ($S_1$, $S_2$, ..., and $S_m$) included in Equation (5) are respectively vectors (spectra). That is, the element $S_1$ is expressed as, for example, Equation (7). The elements ($X_1$, $X_2$, ..., and $X_n$) included in Equation (6) are vectors, and the element $X_j$ is expressed as, for example, Equation (8). The subscript j of the element $X_j$ is the number of wavelength bands obtained by measuring the spectra. The number of elements m of the spectra S of the unknown components is an integer of 1 or more, and is empirically and experimentally determined in advance according to the kind of the sample.

$$S = [S_1, S_2, \cdots, S_m]^T \qquad \cdots (5)$$

$$X = [X_1, X_2, \cdots, X_n]^T \qquad \cdots (6)$$

$$S_i = \{S_{i1}, S_{i2}, \cdots, S_{il}\} \qquad \cdots (7)$$

$$X_i = \{X_{i1}, X_{i2}, \cdots, X_{il}\} \qquad \cdots (8)$$

It is assumed that the respective unknown components are statistically independent. The following relational equation is satisfied between the unknown components S and the spectral data X.

$$X = A \cdot S \qquad \cdots (9)$$

A in Equation (9) is a mixing matrix, and can be expressed as Equation (10). Here, the letter "A" needs to be expressed in boldface as expressed in Equation (10), but is expressed as a normal letter because of the limitation of letters used in the specification. Hereinafter, other boldface letters representing the matrix are similarly expressed as normal letters.

$$A = \begin{pmatrix} a_{11} & \cdots & a_{1m} \\ \vdots & \ddots & \vdots \\ a_{n1} & \cdots & a_{nm} \end{pmatrix} \qquad \cdots (10)$$

A mixing coefficient $a_{ij}$ included in a mixing matrix A represents a degree of contributing the unknown components $S_j$ (j = 1 to m) to the spectral data $X_i$ (i = 1 to n) which is the observational data.

When the mixing matrix A is known, the least squares of the unknown components S may be simply calculated as A+·X by using a pseudo inverse matrix A+ of A. However, in the present exemplary embodiment, since the mixing matrix A is unknown, the unknown component S and the mixing matrix A need to be estimated from only the observational data X. That is, as shown in Equation (11), a matrix (hereinafter, referred to as an "independent component matrix") Y representing the spectra of the independent components is calculated from only the observational data X using mxn separate matrix W. As an algorithm for obtaining the separate matrix W in Equation (11), various algorithms such as Informax, Fast ICA (Fast Independent Component Analysis), and JADE (Joint Approximate Diagonalization of Eigen matrices) may be adopted.

$$Y = W \cdot X \qquad \cdots (11)$$

The independent component matrix Y corresponds to an estimation value of the unknown component S. Thus, Equation (12) can be obtained, and thus, Equation (13) can be obtained by transforming Equation (12).

$$X = \hat{A} \cdot Y \qquad \cdots (12)$$

$$\hat{A} = X \cdot Y^+ \qquad \cdots (13)$$

Here, ^A is an estimation mixing matrix of A, and $Y^+$ is a pseudo inverse matrix of Y.

The estimation mixing matrix ^A (is merely expressed in this manner because of the limitation of the letters used in the specification, and actually means a letter having a symbol thereabove on a left side of Equation (13); the same is true of other letters) obtained in Equation (13) may be expressed in the following equation.

$$\hat{A} = \begin{pmatrix} \hat{a}_{11} & \cdots & \hat{a}_{1m} \\ \vdots & \ddots & \vdots \\ \hat{a}_{n1} & \cdots & \hat{a}_{nm} \end{pmatrix} \qquad \cdots (14)$$

In step S110 of Fig. 10, the CPU 10 performs until the process of obtaining the separate matrix W. Specifically, the separate matrix W is obtained using the spectral data X for each sample which is obtained in Process 2 and is previously stored in the hard disk drive 30 as an input and using any algorithm of Informax, Fast ICA and JADE based on the input. As shown in Fig. 8, it is preferable that the normalization process by the first pre-processing section 450 and the whitening process by the second pre-processing section 460 are performed as the pre-processing of the independent component analysis.

After step S110 is performed, the CPU 10 performs a process of calculating the independent component matrix Y based on the separate matrix W and the spectral data X for each sample which is obtained in Process 2 and is previously stored in the hard disk drive 30 (step S120). In this calculation process, an operation in accordance with Equation (11) is performed. In steps S110 and S120, the CPU 10 functions as the independent component matrix calculating section 432 of Fig. 7.

Subsequently, the CPU 10 performs a process of calculating the estimation mixing matrix ^A based on the spectral data X for each sample that is previously stored in the hard disk drive 30 and the independent component matrix Y calculated in step S120 (step S130). In this calculation process, an operation in accordance with Equation (13) is performed.

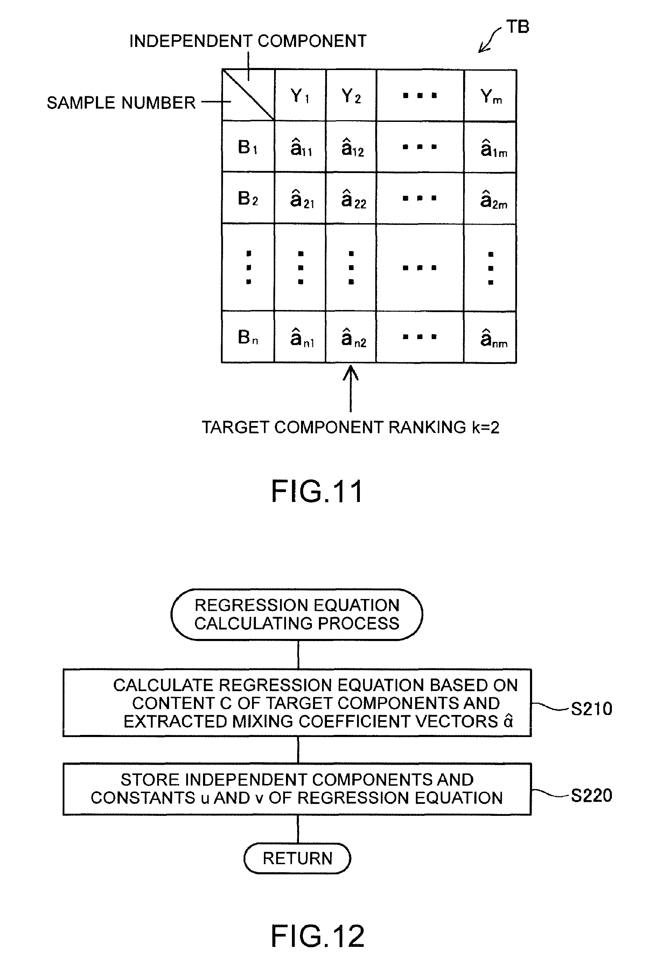

Fig. 11 is an explanatory diagram for describing the estimation mixing matrix ^A. In Table TB, the respective sample numbers $B_1$, $B_2$, ..., and $B_n$ are represented in a vertical direction and the respective elements (hereinafter, referred to as independent component elements") $Y_1$, $Y_2$, ..., and $Y_m$ of the independent component matrix Y are represented in the horizontal direction. In Table TB, the element defined by the sample number $B_i$ (i = 1 to n) and the independent component element $Y_j$ (j = 1 to m) is the same as the element $^a_{ij}$ (see Equation (14)) of the estimation mixing matrix ^A. From Table TB, it can be seen that the element $^a_{ij}$ of the estimation mixing matrix ^A represents a ratio between the independent component elements $Y_1$, $Y_2$, ..., and $Y_m$ in the respective samples. A target component ranking k shown in Fig. 11 will be described. In the process of step S130, the CPU 10 functions as the estimation mixing matrix calculating section 434 of Fig. 7.

Through the processes performed until step S130, the estimation mixing matrix ^A is obtained. That is, the element (estimated mixing coefficient) $^a_{ij}$ of the estimation mixing matrix ^A is obtained. In the example of Fig. 1, the estimated mixing coefficient $^a_{ij}$ corresponds to the inner product value P calculated in (D) to (F) of Fig. 1. Thereafter, the process proceeds to step S140.

In step S140, the CPU 10 obtains the correlation (the degree of similarity) between the target component contents $C_1$, $C_2$, ..., and $C_n$ measured in Process 3 and the components (hereinafter, referred to as mixing coefficient vectors $^\alpha$) of the respective columns included in the estimation mixing matrix ^A calculated in step S130. Specifically, the correlation between the target component contents C ($C_1$, $C_2$, ..., and $C_n$) and the mixing coefficient vectors $^\alpha_1$ ($^a_{11}$, $^a_{21}$, ..., and $^a_{n1}$) of the first column is obtained, and subsequently, the correlation between the target component contents C ($C_1$, $C_2$, ..., and $C_n$) and the mixing coefficient vectors $^\alpha_2$ ($^a_{12}$, $^a_{22}$, ..., and $^a_{n2}$) of the second column is obtained. In so doing, the correlations with respect to the target component contents C for the respective columns are obtained.

As the indicator indicating the magnitude of the correlation, a correlation coefficient R in accordance with the following equation may be used. The correlation coefficient R is also called the Pearson product-moment correlation coefficient.

$$R = \frac{\displaystyle\sum_{i=1}^{n}(C_i - \overline{C})(\hat{a}_{ik} - \overline{\hat{\alpha}_k})}{\sqrt{\displaystyle\sum_{i=1}^{n}(C_i - \overline{C})^2}\sqrt{\displaystyle\sum_{i=1}^{n}(\hat{a}_{ik} - \overline{\hat{\alpha}_k})^2}} \qquad \cdots (15)$$

$\overline{C}$ and $\hat{\alpha}_k$ are respectively target component contents and an average value of elements of vector $^\alpha_k$.

As a result of step S140 of Fig. 10, correlation coefficients $R_j$ (j = 1, 2, ......, and m) for independent components (independent component spectra) $Y_j$ are obtained. Thereafter, the CPU 10 specifies the correlation coefficient of which the correlation is the highest, that is, the value is close to 1 from the correlation coefficient $R_j$ obtained in step S140. The CPU 10 selects a column vector $^\wedge\alpha$ of which the correlation coefficient R is the highest from the estimation mixing matrix $^\wedge$A (step S150).

In Table TB of Fig. 11, the selection in step S150 means that one column is selected from a plurality of columns. Elements of the selected column are mixing coefficients of the independent components corresponding to the target components. As the selected result, the mixing coefficient vector $^\wedge\alpha_k$ ($^\wedge a_{1k}$, $^\wedge a_{2k}$, ..., and $a_{nk}$) is obtained. Here, k is any integer of 1 to m. The value of k may be temporarily stored in the memory 20, as the target component ranking indicating the ranking of the independent component corresponding to the target component. The elements $^\wedge a_{lk}$, $^\wedge a_{2k}$, ..., and $^\wedge a_{nk}$ included in the mixing coefficient vector $^\wedge\alpha_k$ correspond to the "mixing coefficients corresponding to the target component". In the example of Fig. 11, the target component raking k = 2 represents the mixing coefficient vector $^\wedge\alpha_2$ = ($^\wedge a_{12}$, $^\wedge a_{22}$, ..., and $^\wedge a_{n2}$) corresponding to the independent component $Y_2$. In the present specification, the term "ranking" refers to a "value representing a position within the matrix". In the processes of step S140 and S150, the CPU 10 functions as the mixing coefficient selecting section of Fig. 7. After step S150 is performed, the CPU ends the mixing coefficient calculation process. As a result, Process 4 is completed, and subsequently, the process proceeds to Process 5.

Process 5

Process 5 is a regression equation calculation process, and is performed using the computer 100 as in Process 4. In Process 5, the computer 100 performs a process of calculating the regression equation of the calibration curve. The data processed until Process 4 may be transmitted in another computer or device, and Process 5 may be performed using the transmitted data.

Fig. 12 is a flowchart showing the regression equation calculation process performed by the CPU 10 of the computer 100. When the process starts, the CPU 10 calculates the regression equation based on the target component contents C ($C_1$, $C_2$, ..., and $C_n$) measured in Process 3 and the mixing coefficient vectors $^\wedge\alpha_k$ ($^\wedge a_{1k}$, $^\wedge a_{2k}$, ..., and $^\wedge a_{nk}$) selected in step S150 (step S210). The regression equation may be expressed as Equation (16). In step S210, the constants u and v are obtained in Equation (16).

$$C = u \cdot P + v \qquad \cdots (16)$$

Here, C is a target component content, P is an inner product of the measurement data and the independent component, and u and v are constants.

After step S210 is performed, the CPU 10 stores the constants u and v of the regression equation obtained in step S210, and the independent component $Y_k$ corresponding to the target component ranking k (Fig. 11) determined in step S150, as a calibration data set DS2, in the hard disk drive 30 (step S220). Thereafter, the CPU 10 proceeds to "return", and temporarily ends the regression equation calculation process. As a result, it is possible to obtain the regressin equation of the calibration curve, and the calibration curve creation method shown in Fig. 5 is ended. In the processes of step S210 and S220, the CPU 10 functions as the regression equation calculating section 440 of Fig. 7.

D. Calibration Method of Target Component:

A calibration method of the target component will be described. A test object has the same component as the sample used when the calibration curve is created. Specifically, the calibration method of the target component is performed using a computer. Here, the computer may be the computer 100 used when the calibration curve is created, or may be another computer.

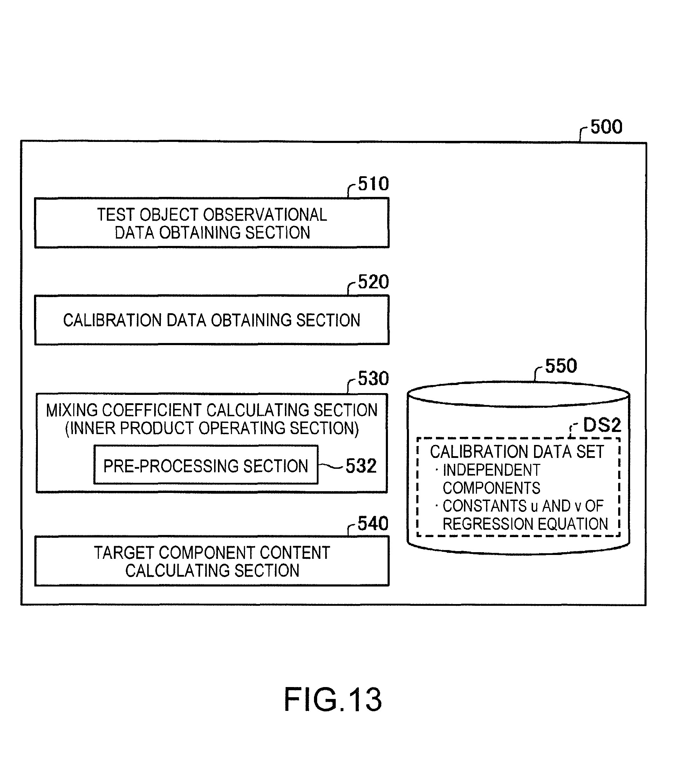

Fig. 13 is a functional block diagram of a device used when the target component is calibrated. A device 500 includes a test object observational data obtaining section 510, a calibration data obtaining section 520, a mixing coefficient calculating section 530, a target component content calculating section 540, and a non-volatile storage device 550. The mixing coefficient calculating section 530 includes a pre-processing section 532. The pre-processing section 532 has a function of both the first pre-processing section 450 and the second pre-processing section 460 of Fig. 8. The mixing coefficient calculating section 530 has a function of performing an inner product operation described in (A) to (C) of Fig. 2, and may be referred to as an "inner product calculating section". The test object observational data obtaining section 510 is implemented by, for example, the CPU 10 of Fig. 6 in cooperation with the input I/F 50 and the memory 20. The calibration data obtaining section 520 is implemented by, for example, the CPU 10 of Fig. 6 in cooperation with the memory 20 and the hard disk drive 30. The mixing coefficient calculating section 530 and the target component content calculating section 540 are implemented by, for example, the CPU 10 of Fig. 6 in cooperation with the memory 20. The calibration data set DS2 (the independent components and the constants u and v of the regression equation) is stored in the non-volatile storage device 550. The device of Fig. 13 may be mounted as another device or electronic device different from the computer of Fig. 6. In this case, it is preferable that the device of Fig. 13 or the electronic device including the device has the spectroscopic measurement device.

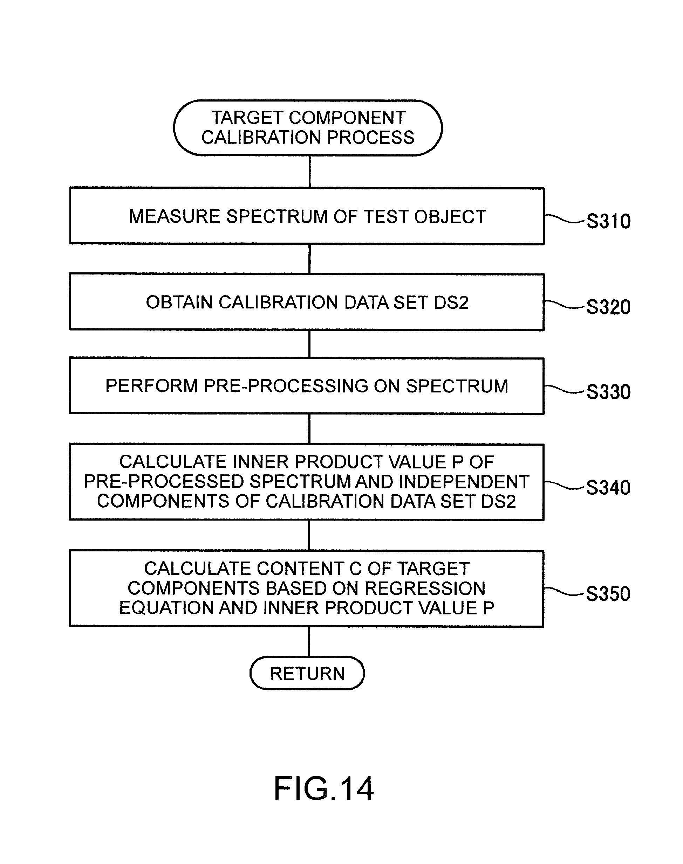

Fig. 14 is a flowchart showing the target component calibration process performed by the CPU 10 of the computer 100. The target component calibration process is implemented in such a manner that the CPU 10 loads a predetermined program stored in the hard disk drive 30 to the memory 20 and executes the loaded program. First, the CPU 10 performs a process of photographing the test object in a spectroscopic measurement device (step S310). The photographing in step S310 may be performed as in Process 2, and as a result, an absorbance spectrum $X_p$ of the test object is obtained. It is preferable that the spectroscopic measurement device used in the calibration process is the same type as the spectroscopic measurement device used to create the calibration curve for controlling the error. In order to further control the error, it is preferable that these processes are performed by the same spectroscopic measurement device. As in Process 2 of Fig. 5, in place of measuring the spectral reflectance spectra and the absorbance spectra in the spectrometer, these spectra may be estimated from other measured values. The absorbance spectrum $X_p$ of the test object obtained when one test object is photographed once is represented by a spectrum as in the following equation.

$$X_p = \{X_{p1}, X_{p2}, \cdots, X_{pl}\} \quad \cdots (1 7)$$

In the process of step S310, the CPU 10 functions as the test object observational data obtaining section 510 of Fig. 13. Subsequently, the CPU 10 obtains the calibration data set DS2 from the hard disk drive 30 (non-volatile storage device 550 of Fig. 13), and stores the obtained data set in the memory 20 (step S320). In the process of step S320, the CPU 10 functions as the calibration data obtaining section 520 of Fig. 13.

After step S320 is performed, the pre-processing is performed on the observational data (absorbance spectrum $X_p$) of the test object obtained in step S310 (step S330). It is preferable that the same process as the pre-processing (that is, the normalization process performed by the first pre-processing section 450 and the whitening process performed by the second pre-processing section 460) used in Process 4 (more specifically, step S110 of Fig. 10) of Fig. 5 when the calibration curve is created is performed as the pre-processing.

Thereafter, the CPU 10 obtains the inner product value P of the independent components included in the calibration data set DS2 and the pre-processed spectrum (the pre-processed observational data) obtained in step S330 (step S340). The process of step S340 corresponds to the processes (B) and (C) of Fig. 2. The inner product value P corresponds to the mixing coefficient calculated in step S130 of Fig. 10 when the calibration curve is created. Accordingly, the inner product value P is also referred to as a "mixing coefficient".

In the processes of Steps S330 and S340, the CPU 10 functions as the mixing coefficient calculating section 530 of Fig. 13.

Subsequently, the CPU 10 calculates the content C of the target component by reading the constants u and v of the regression equation included in the calibration data set DS2 from the hard disk drive 30 (the non-volatile storage device 550 of Fig. 13) and substituting the constants u and v and the inner product value P obtained in step S340 in the right side of Equation (16) (step S350). In this case, the constants u and v may be adjusted when necessary. For example, the content C is obtained as a mass of the target component per unit volume or unit mass of the test object (for example, per 1 dL or per 100 grams). In the process of step S350, the CPU 10 functions as the target component content calculating section 540 of Fig. 13. Thereafter, the process proceeds to "return", and ends the target component calibration process.

Although it has been described in the present exemplary embodiment that the content C obtained in step S350 is the content of the target component of the test object, a value obtained by correcting the content C obtained in step S350 by using a normalization coefficient used for the normalization in step S330 may be used the content to be obtained. Specifically, the absolute value (gram) of the content may be obtained by multiplying the content C by the standard deviation. In such a configuration, it is possible to calculate the content C with higher precision depending on the kind of the target component.

According to the calibration method described above, it is possible to calculate the content of the target component from the spectrum which is an actual value of the test object with high precision.

E. Various Algorithms and Influences thereof:

Hereinafter, various algorithms used in the first pre-processing section 450, the second pre-processing section 460 and the independent component analysis processing section 470 shown in Fig. 8 will be sequentially described.

E-1. First Pre-processing (normalization process using SNV/PNS):

The first pre-processing section 450 may use the SNV (Standard Normal Variate Transformation) and PNS (Project on Null Space) as the first pre-processing.

The SNV is given as the following equation.

$$z = \frac{x - x_{ave}}{\sigma} \qquad \cdots (18)$$

Here, z is processed data, x is processing target data (absorbance spectrum in the present exemplary embodiment), $x_{ave}$ is an average value of the processing target data x, and $\sigma$ is the standard deviation of the processing target data x. As a result of the standard normal variate transformation, normalized data z of which the average value is 0 and the standard deviation is 1 is obtained.

When the PNS is performed, it is possible to reduce the baseline variation included in the processing target data. In the measurement of the processing target data (absorbance spectrum in the present exemplary embodiment), variation between data items which is called the baseline variation such as an increase or decrease in average value of the data for each measurement data occurs due to various factors. For this reason, it is preferable that the variation factors are removed before the ICA (independent component analysis) is performed. The PNS may be used as the pre-processing capable of reducing the baseline variation of the processing target data. Particularly, since such baseline variations frequently occur in the measurement data regarding the reflectance spectrum or the absorbance spectrum including an infrared region, an advantage of applying the PNS is great. Hereinafter, a principle in which the baseline variation included in data (referred to as "measurement data x") obtained through the measurement is removed by the PNS will be described. As a typical example, a case where the measurement data is the reflectance spectrum or the absorbance spectrum including the infrared region will be described. Here, the PNS is similarly applicable to other types of measurement data (for example, voice data).

In general, in an ideal system, the measurement data x (processing target data x) is expressed as the following equation by using the m (m is an integer of 2 or more) independent components $s_i$ (i = 1 to m) and mixing ratios $c_i$ therebetween.

$$x = \sum_{i-1}^{m} c_i s_i \qquad \cdots (19)$$
$$= A \cdot s$$

Here, A is a matrix (mixing matrix) formed using the mixing ratios $c_i$.

In the ICA (independent component analysis), the process is performed on the assumption of this model. However, various variation factors (change in sample state or measurement environment) are present in the actual measurement data. Thus, as the model created by taking into account the variation factors, a model in which the measurement data x is expressed by the following equation is considered.

$$x = b \sum_{i=1}^{m} c_i s_i + aE + b_1 f_1(\lambda) + b_2 f_2(\lambda) + \cdots b_g f_g(\lambda) + \varepsilon \qquad \cdots (20)$$

Here, b is a parameter representing the variation of the spectrum in the amplitude direction, a is a parameter representing the amount of a constant baseline variation E (referred to as an "average value variation"), $b_1$, ..., and bg are parameters representing the amounts of g (g is an integer of 1 or more) variations $f_1(\lambda)$ to $f_g(\lambda)$ depending on the wavelength, and $\varepsilon$ is a variation component other than the above variations. The constant baseline variation E is given as E = {1, 1, 1, ..., and 1}$^T$ (superscript T denotes transposition), and the data length thereof is a constant vector equal to the data length N (a distinct number of a wavelength band) of the measurement data x. As the variable $\lambda$ representing the wavelength, N integers from 1 to N are used. That is, the variable $\lambda$ corresponds to an ordinal number of the data length N (N is an integer of 2 or more) of the measurement data x. In this case, variations $f_1(\lambda)$, ..., and $f_g(\lambda)$ which depend on the wavelength are given as $f_1(\lambda)$ = {$f_1(1)$, $f_1(2)$, ..., and $f_1(N)$ }$^T$, ..., and $f_g(\lambda)$ = {$f_g(1)$, $f_g(2)$, ..., and $f_g(N)$}$^T$. Since these variations are error factors in the ICA or the calibration, it is preferable that these variations are previously removed.

It is preferable that a function of one variable in which the value of the function f ($\lambda$) is monotonously increased along with the increase of $\lambda$ within a $\lambda$ value range of 1 to N is used as a function f($\lambda$). In the projection on null space method, it is possible to further reduce the variation included in the measurement data by using another function other than an exponential function $\lambda\alpha$ of $\lambda$ of which the indicator $\alpha$ is an integer.

As a method of determining a preferred function form of the function f($\lambda$) and the number thereof g, experimental trial and error may be adopted, or an existing parameter estimation algorithm (for example, an expectation-maximization (EM) algorithm) may be used.

In the PNS, it is possible to obtain the data of which baseline variation components E and $f_1(\lambda)$ to $f_g(\lambda)$ are reduced by considering a space including the baseline variation components E and $f_1(\lambda)$ to $f_g(\lambda)$ and projecting the measurement data x in a space (null space) that does not include these variation components. As a specific operation, the data z processed through the PNS is calculated by the following equation.

$$z = \left(1 - PP^+\right)x = b\sum_{i=1}^{m} c_i k_i + \varepsilon^* \qquad \cdots \ (2\,1)$$

$$P = \left\{1, f_1(\lambda), f_2(\lambda) \cdots f_g(\lambda)\right\}$$

Here, P+ is a pseudo inverse matrix. $k_i$ is obtained by projecting an element $s_i$ of Equation (20) into the null space that does not include the variation component. $\varepsilon^*$ is obtained by projecting the variation component s of Equation (20) into the null space.

After the PNS process is performed, when the normalization (for example, SNV) is performed, it is possible to remove an influence of a variation amount b of the spectrum in the amplitude direction in Equation (20).

When the ICA is performed on the pre-processed data through the PNS, the obtained independent component is an estimation value of the component $k_i$ of Equation (21), and is different from the true element $s_i$. However, since the mixing ratio $c_i$ is not changed from the original value of the value in Equation (20), the calibration process (Fig. 2 and Fig. 14) using the mixing ration $c_i$ is not influenced. As discussed above, as the pre-processing of the ICA, when the PNS is performed, since it is difficult to obtain the true elements $s_i$ through the ICA, an idea of applying the PNS to the pre-processing of the ICA may not be suggested. Meanwhile, in the present exemplary embodiment, since the calibration process is not influenced even through the PNS is performed as the pre-processing of the ICA, if the PNS is performed as the pre-processing, it is possible to perform the calibration with higher precision.

The details of the PNS is described in "Extracting Chemical Information from Spectral Data with Multiplicative Light Scattering Effects by Optical Path-Length Estimation and Correction (Zeng-Ping Chen, Julian Morris, and Elaine Martin; 2006)".

E-2. Second pre-processing (whitening process using PCA/FA):

As the second pre-processing performed by the second pre-processing section 460, the PCA (Principal Components Analysis) and the FA (Factor Analysis) may be used.

In a general method of the ICA, the decorrelation and the dimensional compression of the processing target data are performed as the pre-processing. Through the pre-processing, since the transformation matrix to be obtained in the ICA is limited to the orthogonal transformation matrix, it is possible to reduce the calculation amount of the ICA. Such pre-processing is called "whitening", and the PCA is used in many cases. The whitening using the PCA is described in, for example, Chapter 6 of Aapo Hyvarinen, Juha Karhumen, Erkki Oja, "Independent Component Analysis", 2001, John Wiley & Sons, Inc. ("Independent Component Analysis", February 2005, published by Tokyo Denki University Publishing Office).

However, in the PCA, when the random noise is included in the processing target data, an error occurs in the processing result due to the influence of the random noise. Thus, in order to reduce the influence of the random noise, it is preferable that the whitening is performed using the FA (Factor Analysis) having robustness against the noise in place of the PCA. Hereinafter, a principle of the whitening through the FA will be described.

As described, in the ICA, it is assumed that a linear mixing model (Equation (19)) in which the processing target data x is expressed as a linear sum of the elements $s_i$ is used, and the mixing ratios $c_i$ and the elements $s_i$ are obtained. However, random noises other than the elements $s_i$ are added to the actual data in many cases. Thus, a model in which the measurement data x is expressed by the following equation is considered as the model in which the random noise is considered.

$$\mathbf{x} = \mathbf{A} \cdot \mathbf{s} + \rho \qquad \cdots (22)$$

Here, $\rho$ is a random noise.

The whitening in which the noise mixing model is considered is performed, and subsequently, it is possible to estimate the mixing matrix A and the independent components $s_i$ through the ICA.

In the FA of the present exemplary embodiment, it is assumed that the independent components $s_i$ and the random noise $\rho$ follow normal distributions $N(0, I_m)$ and $N(0, \Sigma)$. As generally known, a first parameter $x_1$ of the normal distribution $N(x_1, x_2)$ represents an expectation value, and a second parameter $x_2$ represents a standard deviation. In this case, since the processing target data x is a linear sum of the variables following the normal distribution, the processing target data x also follows the normal distribution. Here, when a covariance matrix of the processing target data x is expressed as V[x], the normal distribution following the processing target data x may be expressed as N(0, V[x]). In this case, a likelihood function regarding the covariance matrix V[x] of the processing target data x may be calculated in the following order.

First, when it is assumed that the independent components $s_i$ are orthogonal to each other, the covariance matrix V[x] of the processing target data x is calculated by the following Equation.

$$V[\mathbf{x}] = E\left[\mathbf{x}\mathbf{x}^T\right] = \mathbf{A}\mathbf{A}^T + \Sigma \qquad \cdots (23)$$

Here, $\Sigma$ is a covariance matrix of the noise $\rho$.

As stated above, the covariance matrix V[x] may be expressed by the mixing matrix A and the covariance matrix $\Sigma$ of the noise. In this case, a log-likelihood function L(A, E) is given by the following equation.

$$L(\mathbf{A}, \Sigma) = -\frac{n}{2}\left\{tr\left(\left(\mathbf{A}\mathbf{A}^T + \Sigma\right)^{-1}C\right) + \log\left(\det\left(\mathbf{A}\mathbf{A}^T + \Sigma\right)\right) + m\log 2\pi\right\} \qquad \cdots (24)$$

Here, n is the data number of data items x, m is the number of independent components, an operator tr is the trace (sum of diagonal elements) of the matrix, and an operator det is a determinant. C is a sample covariance matrix obtained from the data items x through a sample calculation, and is calculated by the following equation.

$$C = \frac{1}{n}\sum_{i=1}^{n}\mathbf{x}_i\mathbf{x}_i^T \qquad \cdots (25)$$

Through maximum-likelihood estimation using the log-likelihood function L(A, E) of Equation (24), it is possible to obtain the mixing matrix A and the covariance matrix $\Sigma$ of the noise. As the mixing matrix A, a mixing matrix that is not nearly influenced by the random noise $\rho$ of Equation (22) is obtained. This is a basic principle of the FA. As the algorithm of the FA, there are various algorithms using algorithms other than the maximum-likelihood estimation. In the present exemplary embodiment, various FAs can be used.

The estimation value obtained through the FA is merely the value of AAT, and when the mixing matrix A appropriate for this value is determined, it is possible to perform the decorrelation on the data while reducing the influence of the random noise, but it is difficult to arbitrarily determine the plurality of elements $s_i$ in order to leave the degrees of freedom of the rotation. Meanwhile, the ICA is a process of reducing the degrees of freedom of the rotation of the plurality of elements $s_i$ such that the plurality of elements $s_i$ is orthogonal to each other. Thus, in the present exemplary embodiment, the values of the mixing matrix A to be obtained through the FA are used as a whitening matrix (whitened matrix), and arbitrariness to the left rotation is specified by the ICA. Thus, after the whitening process robust against the random noise is performed, it is possible to determine the independent elements $s_i$ orthogonal to each other by performing the ICA. As a result of such a process, it is possible to reduce the influence of the random noise, and thus, it is possible to improve the calibration precision regarding the elements $s_i$.

E-3. ICA (kurtosis and P divergence of independence indicator) :

In the ICA (Independent Component Analysis), higher-order statistics indicating the independence between the data items separated using an indicator for separating the independent components is used as the independence indicator. The kurtosis is a typical independence indicator. The ICA using the kurtosis as the independence indicator is described in, for example, Chapter 8 of Aapo Hyvarinen, Juha Karhumen, Erkki Oja, "Independent Comonent Analysis", 2001, John Wiley & Sons, Inc. ("Independent Component Analysis", February 2005, published by Tokyo Denki University Publishing Office).

However, when the outliner such as the spike noise is included in the processing target data, statistics including the outlier is calculated as the independence indicator. For this reason, an error occurs between the original statistics and the calculated statistics of the processing target data, and degradation in separation precision is caused in some cases. Thus, it is preferable that an independence indicator that is not easily influenced by the outlier included in the processing target data is used. As the independence indicator having such characteristics, the $\beta$ divergence may be used. Hereinafter, the principle of the P divergence as the independence indicator of the ICA will be described.

As discussed above, in the ICA, it is assumed that a linear mixing model (Equation (19)) in which the processing target data x is expressed as a linear sum of the elements $s_i$ is used, and the mixing ratios $c_i$ and the elements $s_i$ are obtained. The estimation value y of the element s obtained through the ICA is expressed as y = W·y using the separate matrix W. In this case, the separate matrix W needs to be an inverse matrix of the mixing matrix A.

Here, the log-likelihood function L(^W) of the estimation value ^W of the separate matrix W may be expressed by the following equation.

$$L\left(\hat{\mathbf{W}}\right) = \frac{1}{N} \sum_{t=1}^{N} l\left(\mathbf{x}(t), \hat{\mathbf{W}}\right) \qquad \cdots (2\,6)$$

Here, the element of the summation symbol $\Sigma$ is the log likelihood in each data point x(t). The log-likelihood function L(^W) can be used as the independence indicator in the ICA. The P divergence method is a method of transforming the log-likelihood function L(^W) so as to suppress the influence of the outlier such as the spike noise included in the data by acting a function appropriate for the log-likelihood function L(^W).

When the $\beta$ divergence is used as the independent indicator, the log-likelihood function L(^W) is transformed by the following equation by using a previously selected function $\Phi_\beta$.

$$L_\Phi\left(\hat{\mathbf{W}}\right) = \frac{1}{N} \sum_{t=1}^{N} \Phi_\beta\left(l\left(\mathbf{x}(t), \hat{\mathbf{W}}\right)\right) \qquad \cdots (2\,7)$$

This function $L_\Phi$(^W) is considered as a new likelihood function.

As the function $\Phi_\beta$ for reducing the influence of the outliner such as the spike noise, as the value of the log likelihood (value in parentheses of the function $\Phi_\beta$) becomes smaller, a function in which the value of the function $\Phi_\beta$ is exponentially attenuated is considered. For example, it is possible to use the following function as the function $\Phi_\beta$.

$$\Phi_\beta(z) = \frac{1}{\beta} \{\exp(\beta z) - 1\} \qquad \cdots (2\,8)$$

In this function, as the value of $\beta$ becomes greater, the function value of each data point z (the log likelihood in Equation (27)) becomes smaller. It is possible to empirically determine the value of $\beta$, and it is possible to set the value of $\beta$ to be, for example, about 0.1. The function $\Phi_\beta$ is not limited to the function of Equation (28), and another function such that as the value of $\beta$ becomes greater, the function value of each data point z becomes small may be used.

When the $\beta$ divergence is used as the independence indicator, it is possible to appropriately suppress the influence of the outlier such as the spike noise. When the log-likelihood function $L_\Phi$(^W) expressed by Equation (27) is considered, the pseudorange between probability distributions minimized so as to correspond to the maximization of the likelihood is the $\beta$ divergence. If the ICA using the P divergence as the independence indicator is performed, it is possible to reduce the influence of the outliner such as the spike noise, and thus, it is possible to improve the calibration precision regarding the elements $s_i$.

The ICA using the $\beta$ divergence is described in, for example, Minami Mihoko, Shinto Eguchi, "Robust Blind Source Separation by $\beta$-Divergence", 2002.

F. Modification Examples:

The invention is not limited to the exemplary embodiment and modification examples thereof, and may be implemented in various modes without departing from the gist, and may be modified as follows.

• Modification Example 1:

In the present exemplary embodiment, the number of elements m of the spectrum S of the unknown component is empirically and experimentally determined in advance, but the number of elements m of the spectrum S of the unknown component may be determined by an information criterion known as the minimum description length (MDL) or the Akaike information criteria (AIC) . When the MDL is used, the number of elements m of the spectrum S of the unknown component can be automatically determined through the operation on the observational data regarding the sample. The MDL is described in, for example, "Independent component analysis for noisy data - MEG data analysis, 2000".

• Modification Example 2:

Although it has been described in the aforementioned exemplary embodiment that the test object as a calibration processing target has the same component as the sample used when the calibration curve is created, the test object may include unknown components other than the same component as the sample used when the calibration curve is created. Since it is assumed that the inner product of the independent components is 0, the inner product of independent components corresponding to the unknown components being 0 may be considered, and thus, it is possible to neglect the influence of the unknown components when the mixing coefficients are obtained using the inner product.

• Modification Example 3:

The computer used in the exemplary embodiment may be a dedicated device. For example, the device shown in Fig. 7 or Fig. 13 may be implemented using only the hardware circuit. Alternatively, a part of the functions of the device shown in Fig. 7 or Fig. 13 may be implemented using the hardware circuit, or another part thereof may be implemented using software.

• Modification Example 4:

Although it has been described in the exemplary embodiment that the input of the spectral reflectance spectrum of the sample or the test object is performed by inputting the spectrum measured by the spectroscopic device, the invention is not limited thereto. For example, the optical spectrum may be estimated from a plurality of band images of which the wavelength bands are different, and the estimated optical spectrum may be input. For example, the band images may be obtained by photographing the sample or the test object by a multiband camera including a filter capable of changing a transmission wavelength band.

Among the elements in the aforementioned embodiments and modification examples, the elements other than the elements described in the independent claims maybe additional elements, and may be appropriately omitted.

**Claims**

1. A target component calibration device that calculates the content of target components of a test object, the device comprising:

   a test object observational data obtaining section that obtains observational data regarding the test object;
   a calibration data obtaining section that obtains calibration data which includes independent components corresponding to the target components and a calibration simple regression equation;
   a mixing coefficient calculating section that calculates mixing coefficients of the target components regarding the test object based on the calibration data and the observational data regarding the test object; and
   a target component content calculating section that calculates the content of target components based on the simple regression equation representing the relationship between the content and the mixing coefficients corresponding to the target components, and the mixing coefficients calculated by the mixing coefficient calculating section,
   wherein the target component content calculating section adjusts at least one of two constants of the simple regression equation depending on a measurement condition when the observational data is obtained.

2. The target component calibration device according to claim 1,
wherein the measurement condition is a measurement environment, and
the target component content calculating section adjusts at least one of the two constants of the simple regression equation depending on the measurement environment.

3. The target component calibration device according to claim 1,
wherein the measurement condition is a difference among examinees as the test object, and
the target component content calculating section adjusts at least one of the two constants of the simple regression equation depending on the difference among the examinees.

4. An electronic device including the target component calibration device according to claim 1.

5. An electronic device including the target component calibration device according to claim 2.

6. An electronic device including the target component calibration device according to claim 3.

7. A target component calibration method for calculating the content of target components regarding a test object, the method comprising:

(a) obtaining observational data regarding the test object;
(b) obtaining calibration data including independent components corresponding to the target components and a calibration simple regression equation;
(c) calculating mixing coefficients of the target components regarding the test object based on the calibration data and the observational data regarding the test object; and
(d) calculating the content of target components based on the simple regression equation representing the relationship between the content and the mixing coefficients corresponding to the target components and the mixing coefficients calculated in the obtaining of the mixing coefficients (c),

wherein in the calculating of the content of target components (d), at least one of two constants of the simple regression equation is adjusted depending on a measurement condition when the observational data is obtained.

8. The target component calibration method according to claim 7,
wherein the measurement condition is a measurement environment, and
in the calculating of the content of target components, at least one of the two constants of the simple regression equation is adjusted depending on the measurement environment.

9. The target component calibration method according to claim 7,
wherein the measurement condition is a difference among examinees as the test object, and
in the calculating of the content of target components, at least one of the two constants of the simple regression equation is adjusted depending on the difference among the examinees.

INDEPENDENT COMPONENT ANALYSIS (ICA)

FIG. 1A

FIG. 1B

INDEPENDENT COMPONENT IC1

INDEPENDENT COMPONENT IC2

FIG. 1C

FIG. 1D

FIG. 1E

FIG. 1F

FIG. 2A

ABSORBANCE

1.0
0.7
0.4
900    1000    1100
WAVELENGTH [nm]

PRE-
PROCESSING →

PRE-PROCESSED
WAVEFORM

ABSORBANCE

2.0
0.0
-1.5
900    1000    1100
WAVELENGTH [nm]

FIG. 2B

INNER
PRODUCT
×

INDEPENDENT
COMPONENT IC1

0.04
0.00
-0.10
900    1000    1100
WAVELENGTH [nm]

FIG. 2C

INNER
PRODUCT
VALUE

CALIBRATION CURVE

COMPONENT CONTENT C

$C=uP+v$

INNER PRODUCT
VALUE P

FIG. 2D

ADJUST CALIBRATION CURVE
(REGRESSION EQUATION)
ACCORDING TO MEASUREMENT
CONDITION OF OBSERVATIONAL DATA
→

$C=(k_u \cdot u)P+(k_v \cdot v)$

COMPONENT CONTENT C

C

INNER PRODUCT
VALUE P

FIG. 2E

INNER
PRODUCT
VALUE

BEFORE CALIBRATION CURVE
(REGRESSION EQUATION) IS ADJUSTED

CALIBRATE MEASUREMENT DATA OF 36 °C USING
CALIBRATION REGRESSION EQUATION OF 35 °C

FIG. 3A

AFTER CALIBRATION CURVE
(REGRESSION EQUATION) IS ADJUSTED

CALIBRATE MEASUREMENT DATA OF 36 °C
BY ADJUSTING CONSTANTS OF CALIBRATION
REGRESSION EQUATION OF 35 °C

FIG. 3B

BEFORE CALIBRATION CURVE
(REGRESSION EQUATION) IS ADJUSTED

CALIBRATE MEASUREMENT DATA OF
EXAMINEE B USING CALIBRATION REGRESSION
EQUATION OF EXAMINEE A (NO ADJUSTMENT)

FIG. 4A

AFTER CALIBRATION CURVE
(REGRESSION EQUATION) IS ADJUSTED

CALIBRATE MEASUREMENT DATA OF
EXAMINEE B BY ADJUSTING CONSTANTS OF
CALIBRATION REGRESSION EQUATION OF EXAMINEE A

FIG. 4B

START

PREPARE — PROCESS 1

MEASURE SPECTRUM — PROCESS 2

MEASURE CONTENT OF
TARGET COMPONENTS — PROCESS 3

ESTIMATE MIXING
COEFFICIENTS — PROCESS 4

CALCULATE REGRESSION
EQUATION — PROCESS 5

END

# FIG. 5

FIG. 6

400

410

SAMPLE OBSERVATIONAL DATA
OBTAINING SECTION

420

SAMPLE TARGET COMPONENT
CONTENT OBTAINING SECTION

430

MIXING COEFFICIENT
ESTIMATING SECTION

INDEPENDENT COMPONENT
MATRIX CALCULATING SECTION

432

ESTIMATION MIXING MATRIX
CALCULATING SECTION

434

MIXING COEFFICIENT
SELECTING SECTION

436

440

REGRESSION EQUATION
CALCULATING SECTION

# FIG. 7

432

## INDEPENDENT COMPONENT MATRIX CALCULATING SECTION

450

### FIRST PRE-PROCESSING SECTION (NORMALIZATION PROCESSING SECTION)

452

STANDARD NORMAL VARIATE TRANSFORMATION (SNV)

454

PROJECT ON NULL SPACE (PNS)

460

### SECOND PRE-PROCESSING SECTION (WHITENING SECTION)

462

PRINCIPAL COMPONENTS ANALYSIS (PCA)

464

FACTOR ANALYSIS (FA)

470

### INDEPENDENT COMPONENT ANALYSIS PROCESSING SECTION (ICA PROCESSING SECTION)

472

FIRST PROCESS
( INDEPENDENCE
INDICATOR :
KURTOSIS )

474

SECOND PROCESS
( INDEPENDENCE
INDICATOR :
β DIVERGENCE )

# FIG. 8

DS1

| SAMPLE NUMBER | TARGET COMPONENT CONTENT | SPECTRAL DATA |
|---|---|---|
| $B_1$ | $C_1$ | SPECTRUM $X_1$ |
| $B_2$ | $C_2$ | SPECTRUM $X_2$ |
| ⋮ | ⋮ | ⋮ |
| $B_n$ | $C_n$ | SPECTRUM $X_n$ |

FIG. 9

```
         ╭─────────────────────────────╮
         │    MIXING COEFFICIENT       │
         │    ESTIMATING PROCESS       │
         ╰─────────────────────────────╯
                        │
    ┌───────────────────────────────────────┐
    │   ANALYZE INDEPENDENT COMPONENTS       │
    │     BASED ON SPECTRAL DATA X           │──── S110
    │   (CALCULATE SEPARATE MATRIX W)        │
    └───────────────────────────────────────┘
                        │
    ┌───────────────────────────────────────┐
    │  CALCULATE INDEPENDENT COMPONENT       │
    │  MATRIX Y BASED ON SEPARATE MATRIX W   │──── S120
    │  AND SPECTRAL DATA X (Y = W·X)         │
    └───────────────────────────────────────┘
                        │
    ┌───────────────────────────────────────┐
    │  CALCULATE ESTIMATION MIXING MATRIX Â  │
    │      BASED ON SPECTRAL DATA X AND      │──── S130
    │   INDEPENDENT COMPONENT MATRIX Y       │
    │            (Â = X·Y⁺)                   │
    └───────────────────────────────────────┘
                        │
    ┌───────────────────────────────────────┐
    │  CALCULATE CORRELATION COEFFICIENT R   │
    │    BETWEEN CONTENT C OF TARGET         │
    │   COMPONENTS AND COMPONENTS            │──── S140
    │ (VECTORS â) OF RESPECTIVE COLUMNS      │
    │ INCLUDED IN ESTIMATION MIXING MATRIX Â │
    └───────────────────────────────────────┘
                        │
    ┌───────────────────────────────────────┐
    │  EXTRACT MIXING COEFFICIENT VECTORS â  │──── S150
    │ WITH HIGHEST CORRELATION COEFFICIENT R │
    └───────────────────────────────────────┘
                        │
                 ╭──────────────╮
                 │    RETURN    │
                 ╰──────────────╯
```

The flowchart steps read:

**MIXING COEFFICIENT ESTIMATING PROCESS**

ANALYZE INDEPENDENT COMPONENTS BASED ON SPECTRAL DATA X (CALCULATE SEPARATE MATRIX W) — S110

CALCULATE INDEPENDENT COMPONENT MATRIX Y BASED ON SEPARATE MATRIX W AND SPECTRAL DATA X ($Y = W \cdot X$) — S120

CALCULATE ESTIMATION MIXING MATRIX $\hat{A}$ BASED ON SPECTRAL DATA X AND INDEPENDENT COMPONENT MATRIX Y ($\hat{A} = X \cdot Y^{+}$) — S130

CALCULATE CORRELATION COEFFICIENT R BETWEEN CONTENT C OF TARGET COMPONENTS AND COMPONENTS (VECTORS $\hat{a}$) OF RESPECTIVE COLUMNS INCLUDED IN ESTIMATION MIXING MATRIX $\hat{A}$ — S140

EXTRACT MIXING COEFFICIENT VECTORS $\hat{a}$ WITH HIGHEST CORRELATION COEFFICIENT R — S150

RETURN

# FIG.10

INDEPENDENT COMPONENT

TB

SAMPLE NUMBER

| | $Y_1$ | $Y_2$ | $\cdots$ | $Y_m$ |
|---|---|---|---|---|
| $B_1$ | $\hat{a}_{11}$ | $\hat{a}_{12}$ | $\cdots$ | $\hat{a}_{1m}$ |
| $B_2$ | $\hat{a}_{21}$ | $\hat{a}_{22}$ | $\cdots$ | $\hat{a}_{2m}$ |
| $\vdots$ | $\vdots$ | $\vdots$ | $\cdots$ | $\vdots$ |
| $B_n$ | $\hat{a}_{n1}$ | $\hat{a}_{n2}$ | $\cdots$ | $\hat{a}_{nm}$ |

TARGET COMPONENT RANKING k=2

## FIG.11

REGRESSION EQUATION
CALCULATING PROCESS

CALCULATE REGRESSION EQUATION BASED ON
CONTENT C OF TARGET COMPONENTS AND
EXTRACTED MIXING COEFFICIENT VECTORS â — S210

STORE INDEPENDENT COMPONENTS AND
CONSTANTS u AND v OF REGRESSION EQUATION — S220

RETURN

## FIG.12

500

510

TEST OBJECT OBSERVATIONAL
DATA OBTAINING SECTION

520

CALIBRATION DATA OBTAINING SECTION

550

530

MIXING COEFFICIENT CALCULATING SECTION
(INNER PRODUCT OPERATING SECTION)

PRE-PROCESSING SECTION — 532

DS2

CALIBRATION DATA SET
· INDEPENDENT
  COMPONENTS
· CONSTANTS u AND v OF
  REGRESSION EQUATION

540

TARGET COMPONENT CONTENT
CALCULATING SECTION

FIG.13

```
┌─────────────────────────────────┐
│      TARGET COMPONENT           │
│      CALIBRATION PROCESS        │
└─────────────────────────────────┘
                 │
┌─────────────────────────────────┐
│  MEASURE SPECTRUM OF TEST OBJECT │ ～S310
└─────────────────────────────────┘
                 │
┌─────────────────────────────────┐
│   OBTAIN CALIBRATION DATA SET DS2 │ ～S320
└─────────────────────────────────┘
                 │
┌─────────────────────────────────┐
│  PERFORM PRE-PROCESSING ON SPECTRUM │ ～S330
└─────────────────────────────────┘
                 │
┌─────────────────────────────────┐
│   CALCULATE INNER PRODUCT VALUE P OF │
│ PRE-PROCESSED SPECTRUM AND INDEPENDENT │ ～S340
│  COMPONENTS OF CALIBRATION DATA SET DS2 │
└─────────────────────────────────┘
                 │
┌─────────────────────────────────┐
│    CALCULATE CONTENT C OF TARGET  │
│  COMPONENTS BASED ON REGRESSION   │ ～S350
│ EQUATION AND INNER PRODUCT VALUE P │
└─────────────────────────────────┘
                 │
            ┌─────────┐
            │ RETURN  │
            └─────────┘
```

# FIG.14

Europäisches
Patentamt

European
Patent Office

Office européen
des brevets

# EUROPEAN SEARCH REPORT

Application Number

EP 15 18 7881

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 784 484 A1 (SEIKO EPSON CORP [JP]) 1 October 2014 (2014-10-01) * claims 12,13 * * paragraph [0009] * * paragraphs [0057], [0059], [0168] * * paragraphs [0113] - [0115] * * paragraphs [0163] - [0167] * ----- | 1-9 | INV. G06F17/18 A61B5/145 A61B5/1495 G01N21/27 G01N21/35 |
| A | EP 1 452 135 A1 (MATSUSHITA ELECTRIC WORKS LTD [JP]) 1 September 2004 (2004-09-01) * paragraphs [0025] - [0040], [0044] * * claims 1,9 * ----- | 1-9 | |
| A | US 2012/095303 A1 (HE ZONGYAN [US]) 19 April 2012 (2012-04-19) * paragraphs [0147] - [0155] * ----- | 1-9 | |
| A | US 2003/191377 A1 (ROBINSON MARK RIES [US] ET AL) 9 October 2003 (2003-10-09) * paragraphs [0073] - [0082] * ----- | 1-9 | |

TECHNICAL FIELDS
SEARCHED      (IPC)

G06F
A61B
G01N
G06K
G01K

The present search report has been drawn up for all claims

| Place of search | Date of completion of the search | Examiner |
|---|---|---|
| Berlin | 11 May 2016 | Domingo Vecchioni, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 15 18 7881

This annex lists the patent family members relating to the patent documents cited in the above-mentioned European search report.
The members are as contained in the European Patent Office EDP file on
The European Patent Office is in no way liable for these particulars which are merely given for the purpose of information.

11-05-2016

| Patent document cited in search report | | Publication date | Patent family member(s) | | Publication date |
|---|---|---|---|---|---|
| EP 2784484 | A1 | 01-10-2014 | EP | 2784484 A1 | 01-10-2014 |
| | | | JP | 2014190795 A | 06-10-2014 |
| | | | US | 2014297197 A1 | 02-10-2014 |
| EP 1452135 | A1 | 01-09-2004 | EP | 1452135 A1 | 01-09-2004 |
| | | | JP | 3931638 B2 | 20-06-2007 |
| | | | JP | 2003144421 A | 20-05-2003 |
| | | | US | 2004142402 A1 | 22-07-2004 |
| | | | WO | 03041582 A1 | 22-05-2003 |
| US 2012095303 | A1 | 19-04-2012 | US | 2012095303 A1 | 19-04-2012 |
| | | | WO | 2012051018 A2 | 19-04-2012 |
| US 2003191377 | A1 | 09-10-2003 | AU | 2003224847 A1 | 27-10-2003 |
| | | | EP | 1542581 A2 | 22-06-2005 |
| | | | US | 2003191377 A1 | 09-10-2003 |
| | | | WO | 03087759 A2 | 23-10-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008005920 A
- JP 2001099710 A

**Non-patent literature cited in the description**

- **ZENG-PING CHEN ; JULIAN MORRIS ; ELAINE MARTIN.** *Extracting Chemical Information from Spectral Data with Multiplicative Light Scattering Effects by optical Path-Length Estimation and Correction,* 2006
- **ZENG-PING CHEN ; JULIAN MORRIS ; ELAINE MARTIN.** *Extracting Chemical Information from Spectral Data with Multiplicative Light Scattering Effects by Optical Path-Length Estimation and Correction,* 2006
- **AAPO HYVARINEN ; JUHA KARHUMEN ; ERKKI OJA.** Independent Component Analysis. John Wiley & Sons, Inc, 2001
- Independent Component Analysis. Tokyo Denki University Publishing Office, February 2005
- **AAPO HYVARINEN ; JUHA KARHUMEN ; ERKKI OJA.** Independent Comonent Analysis. John Wiley & Sons, Inc, 2001
- **MINAMI MIHOKO ; SHINTO EGUCHI.** *Robust Blind Source Separation by β-Divergence,* 2002
- *Independent component analysis for noisy data - MEG data analysis,* 2000